# EUROPEAN PATENT SPECIFICATION

(11) **EP 1 575 929 B1**
(45) Date of publication and mention of the grant of the patent: **21.03.2007**
(21) Application number: 03789656.0
(22) Date of filing: 11.12.2003
(51) Int. Cl.: C07D 305/04

(54) **THE METHOD FOR PRODUCTION OF SEMI-FINISHED PRODUCTS USEFUL IN SYNTHESIS OF PACLITAXEL**
VERFAHREN ZUR HERSTELLUNG VON HALBFERTIGEN PRODUKTEN VERWENDBAR FÜR DIE SYNTHESE DES PACLITAXELS
PROCEDE DE PRODUCTION DE PRODUITS SEMI-FINIS UTILES DANS LA SYNTHESE DU PACLITAXEL

(30) Priority: 19.12.2002 PL 35788102
(43) Date of publication of application: 21.09.2005
(73) Proprietor: Agropharm S.A., 95-080 Tuszyn k/kodzi (PL)
(72) Inventor: KOLESINSKA, Beata, PL-23-240 Jozefow n. Wisla (PL); KAMINSKI, J., Zbigniew, PL-94-050 Lodz (PL); KAMINSKA, E., Janina, PL-94-050 Lodz (PL)
(74) Representative: Padée, Grazyna
(86) International application number: PCT/PL2003/000139
(87) International publication number: WO 2004/056790

(56) References cited:
- WO-A-93/06079
- US-A- 5 767 282
- US-A- 6 150 537
- Z. J. KAMINSKI ET. AL.: "2-Chloro-4,6-dimethoxy-1,3,5-triazine. A New Coupling Reagent for Peptide Synthesis." SYNTHESIS , vol. 1987, no. 10, October 1987 (1987-10), pages 917-20, XP001180718
- HONG-WOO LEE ET. AL. : "2-Chloro-4,6-dimethoxy-1,3,5-triazine. A New Effective and Convenient Coupling Reagent for Cephalosporins. " SYNTHETIC COMMUNICATIONS, vol. 28, no. 8, 1998, pages 1339-49, XP009028766

## Description

The matter of the invention is the method for production of semi-finished products useful in synthesis of Paclitaxel. 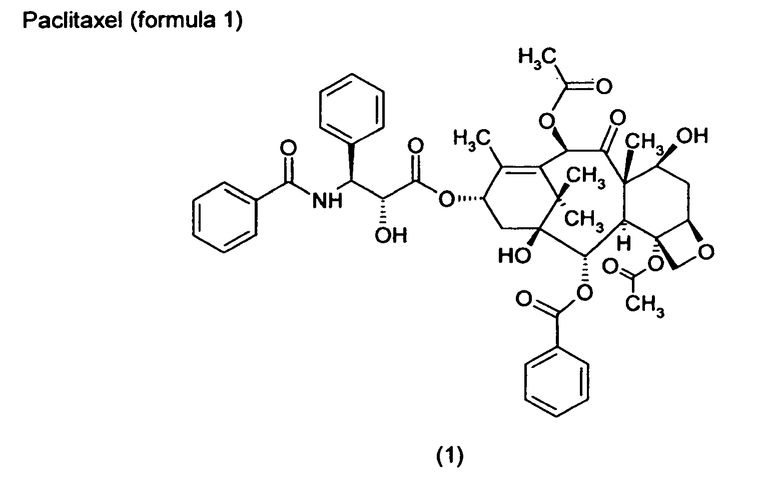 is a natural compound showing vide spectrum of antitumor activity, originally isolated from bark of yew *Taxus brevifolia* [Wani, M.C.; Taylor, H.L.; Wall, M.E.; Coggen, P.; McPhail, A.T., *J. Am. Chem.* Soc., **93,** 2325, (1971)] occurring also in other natural products. Considering low concentrations and difficult regeneration of the natural resources, process of isolation Paclitaxel from plants is of marginal importance. The most effective procedure for production of Paclitaxel is semi-synthesis, based on suitable secured Baccatin III and also secured phenyl isoserine. Baccatin III (formula 3, where R₂=H) 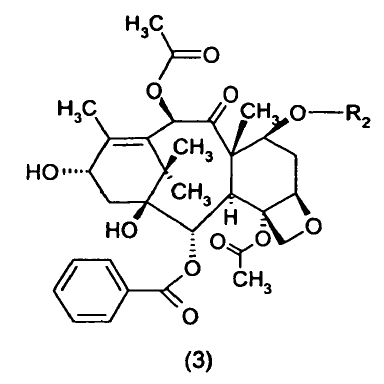 is a heterocyclic compound, characterized by condensed ring arrangement in the [9.3.1.0]^{3,8} pentadecane structure, configured identically as a fragment of Paclitaxel. Baccatin III is obtained from deacetobaccatin II, which occurs in dried needles of yew *Taxus baccata*in concentration approximating to 1 g in 1 kg of the material. Isolation of the substance is an economically sound process, and the resources (needles and shoots) are quite renewable within every one season of vegetation.

All the reported methods for *semi*-synthesis of Paclitaxel are based on the concept of formation of an ester type bound between suitable secured Baccatin III derivative and also secured phenyl isoserine derivatives. However, it should be pointed out, that both the esterification procedure and the product of esterification processing technique depend upon strategy of synthesis and on the type of the protective groups used.

Kingston, D.G.I.; Chaudhary, A.G. Gunatilaka, A.A.L.; Middleton, M.L. in their paper published in *Tetrahedron Lett.,* **35,** 4483-4484 (1994) and in the patent specification WO 97/00870 presented application of dicyclohexylcarboimide (DDC) in presence of 4-(1-pyrrolidin)pyridine for bounding of 7-(triethylsilyl)baccatin [formula 3; R₁= Si(C₂H₅)₃] to 2,4-diphenyl-4,5-dihydrooxazol-5-carboxylic acid, what after removal of the protective groups, provides Paclitaxel at the overall yield of more than 70%.
Similar solution, based on dicyclohexylcarboimide (DDC) in presence of 4-(N,N-dimethylamino)pyridine (DMAP), described by Balogu, E. and Kingston, D.G.I in their paper published in *J. Nat. Prod.* **62,** 1068-1071, (1999) proved to be considerable less effective, because product of condensation yielded 30% yield, only. Moreover, N,N'-dicyclohexylcarboimide was used for acylation of the 7-protected Baccatin derivatives with oxazolidynes and with other phenyl isoserine derivatives.

Drawbacks of all the N,N'- dicyclohexylcarboimide based procedures are difficulties encountered in removal of N,N'- dicyclohexylcarbamide, undesirable byproduct, from the reaction medium. N,N'- dicyclohexylcarbamide is a troublesome impurity of the condensation product because it is sparingly soluble in most of organic solvents, therefore its effective separation from also sparingly soluble Paclitaxel derivatives is difficult. It complicates the process of isolation of intermediates from the final product and reduces effectiveness of utilization of the phenyl isoserine and Baccatin components. Another awkward drawback of DCC is its ability for developing of allergy, manifesting by pathological changes of epidermis. Relatively high vapor pressure of DCC at room temperature results in development of allergic symptoms even in persons, which have no direct contact with the compound.

There are known also other solutions, in which instead of N,N'-dicyclohexylcarboimide other condensing agents are used. Gennori, C.; Garcano, M.; Dughi, M.; Mongelli, N.; Vanatti, E.; Wulpetti, A. in their paper published in *J*. *Org. Chem.* **62**, 4746-4755, (1997), described condensation of 7-(triethylsililo)baccatin with thiophenyl ester of 2,4-diphenyl-4,5-dihydrooxazol-5-carboxylic acid in presence of bis-trimethylsililoamidolite (LHMDS). It yielded 89% of semi-finished product, structure of that was identical to that obtained by Kingston, D. G. et all. with use of N,N'-dicyclohexylcarboimide, as described in *Tetrahedron Lett.,* **35**, 4483-4484 (1994).

Mukaiyama, T. et all. condensed 7-triethylsililobaccatin with phenylisoserine derivative [formula 4c, where R₃=H, R₄=p-C₆H₄OCH₃] in presence of di-(2-pyridil)-thionic carbonate reaching yield of 93%, as published in *Chem. Europ. J.*

Whereas Shiina, I. et all. described in *Chem. Lett.,* **1**, 204 (1998) that condensation of protected Baccatin and phenylisoserine derivative [formula **4b,** where R₂= CH₂C₆H₅] in presence of di-(2-pyridil)-thionic carbonate gives yields 66%, only. Swindell, C. S. et all. in J. *Med. Chem.,* **34,** 1176-1184 (1991) and Greene, A. E. et all. in *J. Am. Chem. Soc.* **110**, 5917 (1998) also described condensation of phenylisoserine derivative with protected Baccatin in presence of dipyridil carbonate and 4-(N,N-dimethylamino)pyridine but the yields reported were moderate.

Patent specification US 6,150,537 provides results of application of acyl chlorides and ketones, obtained from phenylisoserine derivatives, for acylation of the hydroxy group in Baccatin. Considerable drawback of the solution is difficult synthesis of the mentioned phenylisoserine derivatives and the risk of epimerization in course of synthesis of such reactive compounds.

Holton et all. *(J.Am.Chem.Soc,* **116**, 1599-1600, (1994), WO93/06079) in many examples documented suitability of 4-phenyl-3-hydroxyazetydinone-2 derivatives (β-laktam derivatives) for acylation of Baccatin (usually protected in the 7. position with the triethtlsilyl group). Application if the β-laktam derivatives provides the possibility for highly effective acylation, however preparation of these derivatives is a very complex and multistage process.

There are also known applications of phenylisoserine derivatives transformed into a six-membered oxazine ring [US Pat. No 5,015,744, and Beckwermist et all. in *J*. *Org. Chem.,* **61,** 9038-9040 (19996)], however the overall yield of the process is not satisfactory.

According to the invention, the method for bonding of phenylisoserine and baccatine components consists of utilization of an another condensing agent, what effectively eliminates the troubles usually encountered when dicyclohexylcarboimide was used, acylic chlorides, ketenes, thiol esters, di-(2-pyridyl)carbonate, di(2-pyridyl)thiocarbonate, etc.

The method for production of semi-finished products useful in synthesis of Paclitaxel, with the generalized formula **2**, 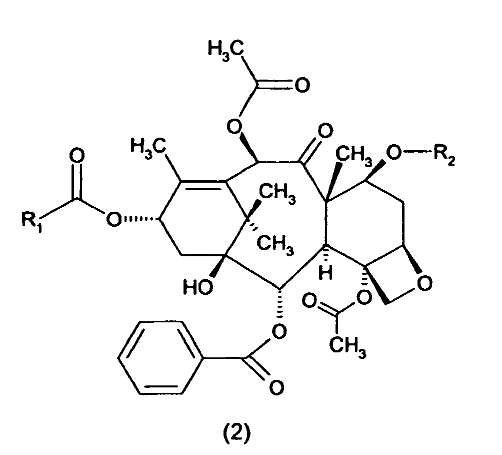 where R₁ denotes a group with formula 7, 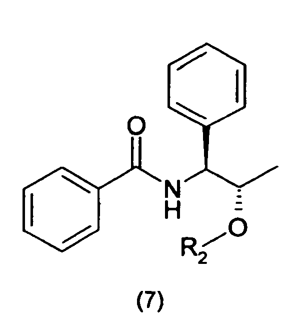 or a group with formula 8, 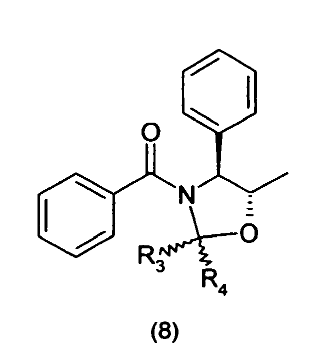 or a group with formula 9, 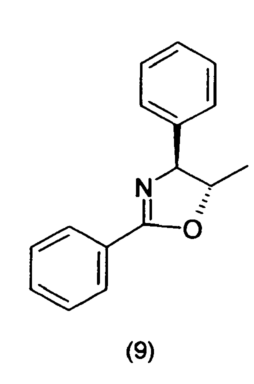 or a group with formula 10,

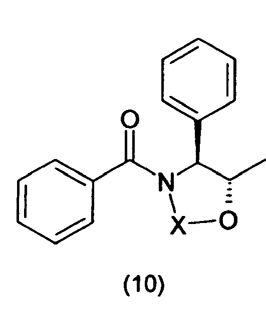

R₂ denotes hydrogen, alkylsilol group or substituted alkylsilol group, arylsilol group or substituted arylsilol group, alkilarylsilol group or substituted alkylarylsilol group, alkoksycarbonyl group or substituted alkoksycarbonyl group, alkoksymethyl group or substituted alkoksymethyl group, R₃ and R₄ denote hydrogen, alkyl group or substituted alkyl group, aryl group or substituted aryl group, and X denotes C=O, C=S, S=O, SO₂, (C=O)₂, P(aryl)₃, P-N(alkyl)₂, (P=O)-N(alkyl)₂, (P=S)-N(alkyl)₂, by reaction of Baccatin III derivatives and phenylisoserine derivatives, consists in reaction of phenylisoserine derivatives, with the generalized formulas 4a-d, 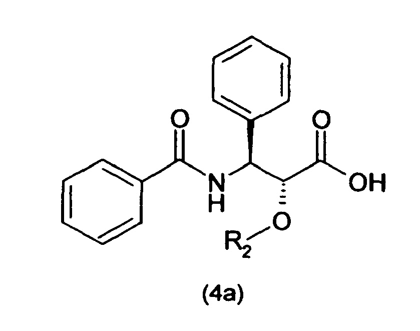 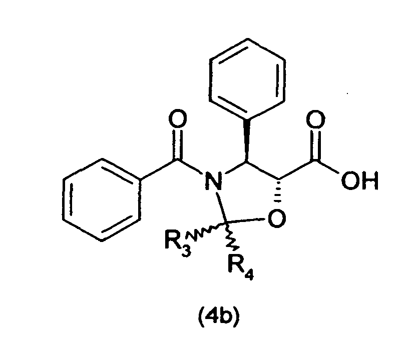 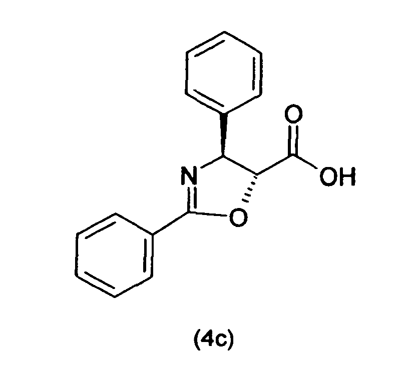 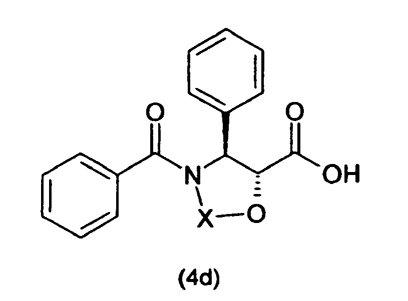 where R₂, R₃, R₄ and X have above specified meanings, or a mixture of their epimers having various configurations in the carbon 2', or their salts, with triazin based condensing agent with the generalized formula 5, 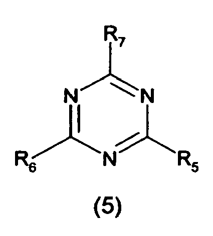 where R₅ and R₆ denote chlorine, alkoxy group, substituted akoxy group, aryloxy group or substituted aryloxy group, whereas R₇ denotes fluorine, chlorine or a quartemary ammonia group, possible in the presence of a tertiary amine group, in anhydrous organic solvent medium, foloowed by reaction of such obtained triazine esters of phenylisoserine, with the generalized formulas 6a-d, 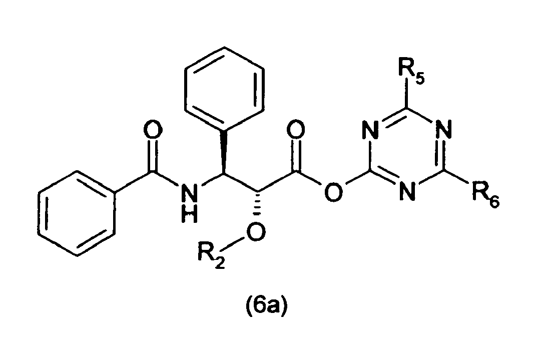 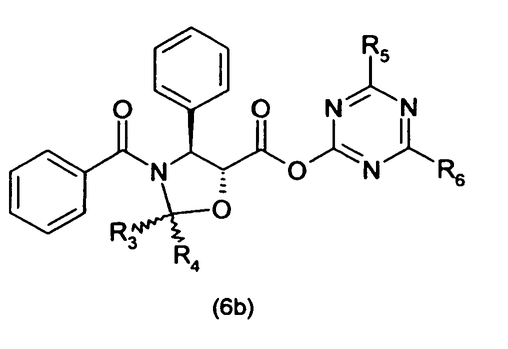 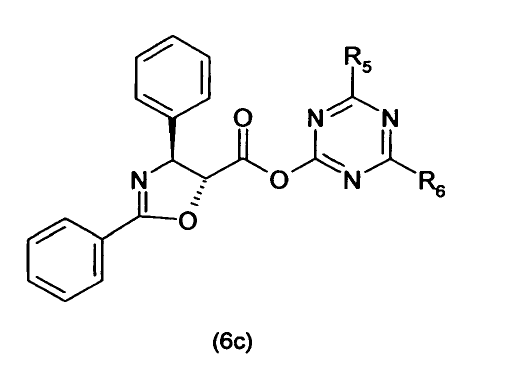 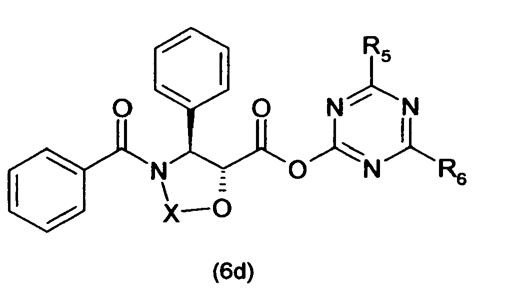 where R₂, R₃, R₄ and X have above specified meanings, with Baccatin with the generalized formula 3, 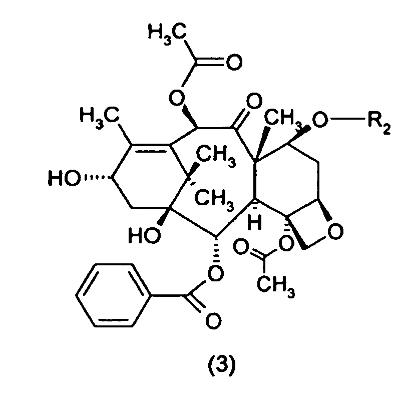 where R₂ denotes hydrogen or a group which protects functional hydroxy group in the position 7., in anhydrous organic solvent, if need be in presence of a catalyst.

Beneficially, phenyl isoserine derivatives are used, in which R₃ and R₄ denote hydrogen, methyl group, phenyl group, metoxyphenyl group or dimetoxy phenyl group.

Beneficially, the hydroxyl group in the 7. position of Baccatin III is protected by alkylsilol group or substituted alkylsilol group, arylsilol group or substituted arylsilol group, alkilarylsilol group or substituted alkylarylsilol group, alkoksycarbonyl group or substituted alkoksycarbonyl group, alkoksymethyl group or substituted alkoksymethyl group.

Beneficially, salts of phenyl izoserine are salts of metals, particularly of alkali metals or salts of tertiary amines, beneficially of N-methylmorpholine, N,N-dimethylaminopyridyne or 1,8-diazabicyclo[5.4.0.]undec-7-ene (DBU).

Beneficially, as the triazine based condensing reagent there is used 2-chloro-4,6-dimethoxy-1,3,5-triazine or quarternary triazinyl ammonium salts, obtained by reaction of 2-chloro-4,6-dimethoxy-1,3,5-triazine with ternary amines.

Beneficially, the tertiary amine is N-methylmorpholine, tetramethylguanidyne, 1,4-dimethylpiperazyne, N-methylpyrolidyne, N,N'-tetramethylethylodiamine, N-methylpiperidine, pyridine, pyridine derivatives substitutes in the 4. Position of the pyridine ring, 1,8-diazabicyclo[5.4.0.]undec-7-en.

Beneficially, the solvent is an a-proton solvent, particularly aromatic hydrocarbons, chlorinated hydrocarbons, ethers, acid amides, nitriles of acids. Particularly beneficial is application of toluene, dichlormethane, dichlorethane, tetrahydrofurane, ethyl acetate, N,N-dimethylformamide, N,N-dimethylacetamide and N,N',N'-hexamethylamide of phosphoric acid, acetonitrile.

Beneficially, the reaction of acylation of Baccatin III proceeds in presence of Lewis-type acid and particularly in presence of anhydrous magnesium bromide.

Beneficially, reaction of acylation of Baccatin III is accomplished in presence of a base, particularly of tertiary amine, and the best in presence of 4-(N,N-dialkylamino)pyridine or DBU.

If the need be, triazine esters of phenyl isoserine may be removed from the reaction medium, before reaction with Baccatin III.

Next, the semi-finished products obtained with the method of invention are transformed into Paclitaxel with known methods, selected accordingly to the nature of the protective group used in reaction of Baccatin and phenyl isoserine. These known methods for transformation of the semi-finished products obtained with the method of invention into Paclitaxel are described e.g. in the publication "Protective Group in Organic Synthesis", ed.2, J. Wiley and Son, 1991.

Triazine type condensing reagents are easily available, of low toxicity and not develop of allergy. They activate the carboxylic function in mild conditions, and process of acilation of the carboxyl function may be accelerated by introduction of suitable catalysts, if required. Both the triazine type condensing reagents, triazine esters and all by-products formed are weak bases in character and after the end of the process, may be readily removed by washing out with diluted acid solutions. The fact makes purification of Paclitaxel and its derivatives, easier and is conductive to high yield of the process.

Very reactive triazine esters my be isolated from the mixture but more convenient is synthesis directly before, or in course of coupling of the phenyl isoserine and the Baccatin components. Such procedure is advantageous, because most of the phenyl isoserine derivatives suitable for *semi-*synthesis of Paclitaxel and the triazine condensation agents are stable and may be easily stored, without any decomposition trace until will be used for coupling with suitable protected Baccatin III.
The method of invention is illustrated by following practicable examples.

### 1. Example

Solution of 2-chloro-4,6-dimethoxy-1,3,5-triazyne (CDMT) (7.71 g, 44 mmoles) in 100 ml THF was mixed with N-methylmorpholine (4.84 ml, 44 mmoles) for 30 minutes at temperature of 0-5°C. Then, there was added mixture of diastereomeres of the **4b** acid, where R₃=H and R₄=3,4-dimethoxyphenyl (19.05 g, 44 mmoles) and further mixed for 4 hours at temperature of 0-5°C, followed by mixing for 8 hours at room temperature. Precipitate was filtered off and to the filtrate (without separation of triazine ester) protected Baccatin III of the 3. formula, where R₂=Si(C₂H₅)₃ (28.1g, 40 mmoles), DMAP (0.24 g, 2 mmoles) MgBr₂ (0.36 g, 2 mmoles) and triethylamine (3.84 g, 38 mmoles) were added. After reaction was completed, solvent was removed under reduced pressure, and the residue was dissolved in ethyl acetate and washed in turn with: H₂O, 1 M KHSO₄, H₂O, 1M NaHCO₃, H₂O by 20 ml each. TLC: (acetone : benzene 1:4) R_{f}=0.75 and 0.8. The process yielded 44.6 g (quantitative yield) of the product with the formula **2**, where R₁ is defined with the formula 8, R₂ is the triethylsilol group, R₃=H, and R₄=3,4-dimethoxyphenyl. Raw product of condensation was dissolved in the mixture methanol/THF (80 ml/450 ml, respectively) and for 40 minutes treated with concentrated hydrochloric acid (30 ml). The mixture was poured into ice water and ethyl acetate mix, the layers separated, then the water phase was extracted with ethyl acetate. The organic phases were connected together and washed with saturated water solution of sodium bicarbonate, dried with magnesium sulfate and concentrated under reduced pressure until dry. Dry residue was chromatographed in a column filled with silica gel. Finally, the product was eluted with the mixture of ethyl acetate/hexane 1:1 (v/v). It gave 28.5 g Paclitaxel with melting point of 221-223°C. The yield amounted to 82%.
[a]_{D}²⁰ = -49 (c=0.45, MeOH).
¹H-NMR: (250 MHz, CDCl₃): δ=1.14 (s, 3H); 1.24 (s, 3H); 1.69 (s, 3H); 1.79 (s, 3H), 1.82 (s, 1 H); 1.88 (m, 1H); 2.24 (s, 3H), 2.25-2.40 (m, 2H); 2.39 (s, 3H); 2.49 (d, J=4.0 Hz, 1H), 2.50-2.55 (m, 1 H); 3.59 (d, J=5.0 Hz, 1H); 4.17 (d, J=9.0 Hz, 1 H); 4.29 (d, J=9.0 Hz, 1 H); 4.37-4.40 (m, 1 H); 4.75-4.79 (m, 1 H); 4.95 (m, 1H); 5.67 (d, J=7.0 Hz, 1 H); 5.77-5.80 (m, 1H); 6.22 (t, J=8.0 Hz, 1 H); 6.25 (s, 1H); 7.05 (d, J=9.5 Hz, 1H); 7.31-7.55 (m, 7H); 7.61-7.65 (m, 1H); 7.71-7.75 (m, 2H); 8.09-8.15 (m, 2H) [ppm]
¹³C-NMR: (250 MHz, CDCl₃): δ=9.4; 15.0; 20.8; 21.7; 22.6; 26.7; 35.5; 35.5; 43.2; 45.5; 55.1; 58.7; 72.2; 72.4; 73.3; 75.0; 75.5; 76.6; 79.1; 81.2; 84.5; 127.2; 127.4;128.5; 138.8; 128.8; 129.1; 129.2; 130.3; 132.1; 133.1; 133.7; 133.8; 133.9, 142.0; 167.1; 167.1; 170.7; 171.5; 172.9; 203.7 [ppm].
IR: 1240, 1550, 166,0; 2970; 3480 [cm⁻¹].
FAB-MS: 854 [MH]⁺; 794 [MH-AcOH]⁺, 776 [M-AcOH-H₂O]⁺.
Cl(isobutane) MS: 854 [MH]⁺; 836 [MH-H₂O]; 818 [MH-2H₂O]⁺.

### 2. Example

N-(4,6-dimethoxy-1,3,5-triazin-2-ylo)-N-methylmorpholine chloride (0.278 g, 1 mmole), cooled down to temperature of 0-5°C in 5 ml chloroform was connected with mixture of epimerides of the **4b** acid, where R₃=H and R₄=3,4-dimethoxyphenyl (0.433 g, 1 mmole) and mixed for 12 hours. N-methylmorpholine chloride was washed off with ice water, and organic phase was dried with MgSO₄, the drying agent was filtered off and the filtrate was evaporated under reduced pressure until dry. It gave respective triazine ester of the formula **6b,** where R₃=H, R₄=3,4-dimethoxyphenyl, and R₅=R₆=OCH₃, with the quantitative yield.
TLC: eluent CHCl₃, R_{f}=0.55
¹H-NMR (250 MHz, CDCl₃): δ= 3.54 (s, 6H); 3.80 (s, 3H); 3.85 (s, 3H); 5.34 (d, J=, 1H); 6.19 (d, J=, 1H), 6.74 (bs, 1H); 7.07 (bs, 1 H); 7.14 (bs, 1H); 7.21 (bs, 1H); 7.28-7.80 (m, 10H) [ppm]
IR (CCl₄): 1750 [cm⁻¹]

The triazine ester was used for reaction with protected Bacctain of the generalized formula **3**, where R₂=Si(C₂H₅)₃ (28.1g, 40 mmoles), DMAP (6 µg, 0.05 mmole) MgBr₂ (9 µg, 0.05 mmole) and triethylamine (96 µg, 0.95 mmole). After the reaction was finished, solvent was removed by evaporation under reduced pressure whereas the residue was dissolved in ethyl acetate and washed in turn with: H₂O, 1M KHSO₄, H₂O, 1 M NaHCO₃, H₂O by 20 ml each. TLC: (acetone : benzene 1:4) R_{f}=0.75. 0.8. It gave 1.1 g (yield 95%) of the product with the formula **2**, where R₁ is defined with the formula 8, R₂= Si(C₂H₅)₃, R₃=H, and R₄=3,4-dimethoxyphenyl.
¹H-NMR: (250 MHz, CDCl₃): δ=0.51 (q, J=8.0 Hz, 6H); 1.23 (s, 3H); 1.22 (t, J=8.0 Hz, 9H); 1.35 (s, 3H); 1.85 (s, 1 H); 1.95 (s, 3H); 2.08 (s, 3H); 2.14 (s, 3H); 2.16-2.28 (m, 1 H); 2.41-2.45 (m, 1H), 2.55-2.58 (m, 1H); 2.59 (s, 3H); 2.65-2.85 (m, 1 H); 3.28 (s, 3H); 3.38 (s, 3H); 4.25 (d, J=7.0 Hz, 1 H); 4.41 (d, J=9.0 Hz, 1 H); 4.48 (d, J=9.0 Hz, 1H); 4.85 (s, 1 H); 4.92-4.96 (m, 1 H); 5.07 (d, J=8.5Hz, 1 H); 6.02 (s, 1 H); 6.15 (d, J= 7.5Hz, 1 H); 6.62-6.68 (m, 1 H); 6.85-6.95 (m, 3H), 6.98-7.08 (m, 2H); 7.12-7.38 (m, 7H); 7.39-7.45 (m, 2H); 7.51 (s, 1 H); 7.61 (d, J=7.5Hz, 1H); 7.77-7.87 (m, 2H); 8.28-8.35 (m, 2H) [ppm]

### 3. Example

Solution of 2-chlor-4,6-dimethoxy-1,3,5-triazine (CDMT) (0.175 g, 1 mmole) in 5 ml THF was mixed with N-methylmorpholine (0.11 ml, 1 mmole) for 30 minutes at temperature of 0-5°C. Then, easily migrated epimeride **4b**, where R₃=H, and R₄=3,4-dimethoxyphenyl (0.433 g, 1 mmol) was added and mixed for 12 hours. N-methylmorpholine chloride was filtered off, and filtrate was evaporated until dry under reduced pressure. It gave respective triazine ester of the formula **6b**, where R₃=H, R₄=3,4-dimethoxyphenyl, and R₅=R₆=OCH₃, with the quantitative yield.
TLC: eluent CHCl₃, R_{f}=0,7.
¹H-NMR (250 MHz, CDCl₃): δ= 3.52 (s, 6H); 3.80 (s, 3H); 3.85 (s, 3H); 5.34 (d, J=, 1H); 6.19 (d, J=, 1H), 6.74 (bs, 1H); 7.07 (bs, 1H); 7.11 (bs, 1H); 7.21 (bs, 1H); 7.28-7.80 (m, 10H) [ppm]
IR (CCl₄): 1750 [cm⁻¹]

The triazine ester, in the presence of 4-(pyrolidin)pyridine, was coupled with protected Bacctain of the generalized formula **3**, where R₂=Si(C₂H₅)₃. After the reaction was finished, raw product was extracted as described in the 1. Example. After removal of protective groups and chromatography it gave Paclitaxel with melting point of 220-223°C and chromatographic and spectral characteristics identical to those described in the 1. Example.

### 4. Example

Solution of 2-chlor-4,6-dimethoxy-1,3,5-triazine (CDMT) (0.175 g, 1 mmole) in 5 ml THF was mixed with N-methylmorpholine (0.11 ml, 1 mmole) for 30 minutes at temperature of 0-5°C. Then, hardly migrating epimeride 4b, where R₃=H, and R₄=3,4-dimethoxyphenyl (0.433 g, 1 mmol) was added and mixed for 12 hours. N-methylmorpholine chloride was filtered off, and filtrate was evaporated until dry under reduced pressure. It gave respective triazine ester of the formula 6b, where R₃=H, R₄=3,4-dimethoxyphenyl, and R₅=R₆=OCH₃, with the quantitative yield.
TLC: eluent CHCl₃, R_{f}=0.6
¹H-NMR (250 MHz, CDCl₃): δ= 3.56 (s, 6H); 3.80 (s, 3H); 3.85 (s, 3H); 5.34 (d, J=, 1H); 6.19 (d, J=, 1H), 6.74 (bs, 1H); 7.07 (bs, 1 H); 7.14 (bs, 1 H); 7.21 (bs, 1H); 7.28-7.80 (m, 10H) [ppm]
IR (CCl₄): 1740 [cm⁻¹]

The triazine ester, in the presence of magnesium bromide and 4-(pyrolidin)pyridine, was coupled with protected Bacctain III of the generalized formula 3, where R₂=Si(C₂H₅)₃-After the reaction was finished, raw product was extracted as described in the 1. Example. After removal of protective groups and chromatography it gave Paclitaxel with melting point of 220-223°C and chromatographic and spectral characteristics identical to those described in the 1. Example.

### 5. Example

Solution of 2-chlor-4,6-diphenoxy-1,3,5-triazine (CDPhT) (0.3 g, 1 mmole) in 5 ml THF was mixed with N-methylmorpholine (0.11 ml, 1 mmole) for 30 minutes at temperature of 0-5°C to obtain N-(4,6- diphenoxy -1,3,5-triazine-2-yl)-N- methylmorpholine chloride. Then, mixture of epimerides of acids **4b**, where R₃=H, and R₄=3,4-dimethoxyphenyl (0.433 g, 1 mmol) was added and mixed for 12 hours. After N-methylmorpholine chloride was filtered off, and filtrate was evaporated it gave respective triazine ester of the formula **6b**, where R₃=H, R₄=3,4-dimethoxyphenyl, and R₅=R₆=OCH₃, with the quantitative yield.
TLC: eluent CHCl₃, R_{f}=0.45,
¹H-NMR (250 MHz, CDCl₃): δ=3.80 (s, 3H); 3.85 (s, 3H); 5.34 (d, J=, 1 H); 6.19 (d, J=, 1H), 6.74 (bs, 1 H); 7.07 (bs, 1 H); 7.09 (bs, 1 H); 7.10 (bs, 1 H); 7.11-7.76 (m, 20H) [ppm]
IR (KBr): 1770 [cm⁻¹]

The triazine ester, in the presence of magnesium bromide and 4-(pyrolidin)pyridine, was coupled with protected Bacctain III of the generalized formula **3**, where R₂=Si(C₂H₅)₃. The product was chromatographically and spectrally identical to that described in the 2. Example.

### 6. Example

Solution of 2,4-dichlor-1,3,5-triazine (DCMT) (0.09 g, 0.5 mmole) in 5 ml THF was mixed with N-methylmorpholine (0.11 ml, 1 mmole) for 30 minutes at temperature of 0-5°C to form respective quaternary triazinammonium salt. Then, mixture of epimerides **4b,** where R₃=H, and R₄=3,4-dimethoxypheny) (0.433 g, 1 mmol) was added and mixed for 12 hours, first at temperature of 0-5°C next at room temperature. N-methylmorpholine hydrochloride was filtered off, and solvent from filtrate was evaporated. It gave respective triazine ester of the formula 6b, where R₃=H, R₄=3,4-dimethoxyphenyl, and R₅=OCH₃, with the quantitative yield.
TLC: eluent CHCl₃, R_{f}=0.45
¹H-NMR (CDCl₃): δ=3.63 (s, 3H); 3.80 (s, 3H); 3.85 (s, 3H); 5.34 (d, J=, 1H); 6.19 (d, J=, 1 H), 6.74 (bs, 1 H); 7.07 (bs, 1H); 7.14 (bs, 1H); 7.21 (bs, 1 H); 7.28-7.80 (m, 10H) [ppm]
IR (KBr): 1760 [cm⁻¹]

The triazine ester, in the presence of magnesium bromide, was coupled with protected Baccatin III of the generalized formula 3, where R₂=Si(C₂H₅)₃. The product was chromatographically and spectrally identical to that described in the 2. Example.

### 7. Example

Solution of 2-chlor-4,6-dimethoxy-1,3,5-triazine (CDMT) (0.175 g, 1 mmole) in 5 ml acetonitrile was mixed with tetramethyloguanidyne (0.126 ml, 1 mmole) for 30 minutes at temperature of 0-5°C. Then, mixture of epimerides of acids **4b,** where R₃=H, and R₄=3,4-dimethoxyphenyl (0.433 g, 1 mmol) was added and mixed for 12 hours. Tetramethyloguanidyne hydrochloride was washed off with ice water, filtrate was dried over MgSO₄, next the drying agent was filtered off and the residue was evaporated until dry under reduced pressure. It gave respective triazine ester of the formula 6b, where R₃=H, R₄=3,4-dimethoxyphenyl, and R₅=R₆=OCH₃, with the quantitative yield.
The triazine ester, in the presence of magnesium bromide, was coupled with protected Bacctain III of the generalized formula **3,** where R₂=Si(C₂H₅)₃. The product was chromatographically and spectrally identical to that described in the 2. Example.

### 8. Example

Solution of 2-chlor-4,6-dimethoxy-1,3,5-triazine (CDMT) (0.175 g, 1 mmole) in 5 ml toluene was mixed with 1,4-dimethylpiperazyne (0.135 ml, 1 mmole) for 30 minutes at temperature of 0-5°C. Then, mixture of epimerides of acids **4b,** where R₃=H, and R₄=3,4-dimethoxyphenyl (0.433 g, 1 mmol) was added and mixed for 12 hours. 1,4-dimethylpiperazyne hydrochloride was filtered off, filtrate was evaporated until dry under reduced pressure. It gave respective triazine ester of the formula **6b,** where R₃=H, R₄=3,4-dimethoxyphenyl, and R₅=R₆=OCH₃, with the quantitative yield.
The triazine ester, in the presence of 4-(N,N-dimethylamin)pyridyne, was coupled with protected Bacctain III of the generalized formula 3, where R₂=Si(C₂H₅)₃. The product was chromatographically and spectrally identical to that described in the 2. Example.

### 9. Example

Solution of 2-chlor-4,6-dimethoxy-1,3,5-triazine (CDMT) (0.175 g, 1 mmole) in 5 ml DMF was mixed with N-methylpiperidine (0.104 ml, 1 mmole) for 30 minutes at temperature of 0-5°C. Then, mixture of epimerides of acids **4b,** where R₃=H, and R₄=3,4-dimethoxyphenyl (0.433 g, 1 mmol) was added, mixed for 12 hours and evaporated until dry under reduced pressure. After washing off, respective triazine ester of the formula 6b, where R₃=H, R₄=3,4-dimethoxyphenyl, and R₅=R₆=OCH₃ was obtained with the quantitative yield. The triazine ester, in the presence of magnesium bromide and 4-(N,N-dimethylamin)pyridyne, was coupled with protected Bacctain III of the generalized formula **3,** where R₂=Si(C₂H₅)₃. The product was chromatographically and spectrally identical to that described in the 2. Example.

### 10. Example

Solution of 2-chlor-4,6-dimethoxy-1,3,5-triazine (CDMT) (0.175 g, 1 mmole) in 5 ml dichloroethane was mixed with N-methylpyrolidyne (0.104 ml, 1 mmole) for 30 minutes at temperature of 0-5°C. Then, mixture of salts of epimerides of acids **4b,** where R₃=H, and R₄=3,4-dimethoxyphenyl (0.455 g, 1 mmol) was added and mixed for 12 hours. After washing off and evaporation of solvent, respective triazine ester of the formula 6b, where R₃=H, R₄=3,4-dimethoxyphenyl, and R₅=R₆=OCH₃ was obtained with the quantitative yield.

The triazine ester, in the presence of magnesium bromide and 4-(N,N-dimethylamin)pyridyne, was coupled with protected Bacctain III of the generalized formula 3, where R₂=Si(C₂H₅)₃. The product was chromatographically and spectrally identical to that described in the 2. Example.

### 11. Example

Solution of 2-chlor-4,6-dimethoxy-1,3,5-triazine (0.175 g, 1 mmole) in 5 ml THF was mixed with N-methylmorpholine (0.11 ml, 1 mmole) for 30 minutes at temperature of 0-5°C, to form respective quaternary triazinilammonium salt. Then, phenyl isoserine of the formula **4b,** where R₃= R₄=CH₃ (0.325 g, 1 mmol) was added and mixed for 12 hours, first at temperature of 0-5°C, next at room temperature. N-methylmorpholine was filtered off and solvent was removed from the filtrate. It gave respective triazine ester of the formula **6b**, where R₃ =R₄=CH₃, R₅=R₆=OCH₃, with the quantitative yield.
TLC: eluent CHCl₃, R₁=0.50
¹H-NMR (CDCl₃): δ= 1.76 (s, 3H); 1.79 (s, 3H); 3.76 (s, 6H); 5.20 (d, J= 7.5 Hz, 1H); 5.59, (d, J= 7.5 Hz, 1H); 7.43-7.56 (m, 8H); 7.70-7.43 (m, 2H) [ppm].
IR (KBr): 1790 [cm⁻¹]
The triazine ester was coupled with protected Baccatin III of generalized formula **3,** where R₂=Si(C₂H₅)₃, in the presence of magnesium bromide and diazobicycloundekane. It gave 0.945 g of the final product (93.8%) with the melting temperature of 171-173°C.
TLC: eluent (acetone:benzene 1:4), R_{f}=0.85
¹H-NMR (CDCl₃): δ=0.55 (q, J=8.5 Hz, 6H); 0.94 (t, J=8.5 Hz, 9H); 1.17 (s, 3H); 1.22 (s, 3H); 1.67 (s, 3H); 1.77 (s, 1H); 1.83-1.85 (m, 1H); 1.87 (s, 3H); 1.94 (s, 3H); 2.02 (s, 3H); 2.07 (s, 3H); 2.02-2.18 (m, 2H); 2.19 (s, 3H); 2.07-2.20 (m, 2H); 2.48-2.50 (m, 2H); 3.77 (d, J=7.5 Hz, 1H); 4.07 (d, J=8.5 Hz, 1H); 4.25 (d, J=8.5 Hz, 1H); 4.42-4.46 (m, 1H); 4.58 (d, J=7.5 Hz, 1 H); 4.88-4.93 (m, 1 H); 5.28 (d, J=6.5 Hz, 1 H); 5.68 (d, J=7.5 Hz, 1H); 6.21-6.25 (m, 1H); 6.49 (s, 1 H); 6.91-6.96 (m, 1H); 7.07-7.22 (m, 8H); 7. 44-7.48 (m, 2H); 7.61-7.65 (m, 1 H); 8.02 (d, J=7.5 Hz, 2H) [ppm]

The product of condensation was treated with formic acid in THF, and the final product was chromatographically purified on silica gel with the system of hexane: ethyl acetate 2:1. It gave 0.621 g (yield 76%) of a product spectroscopically and chromatographically identical to Paclitaxel.

### 12. Example

Solution of 2-chlor-4,6-diphenoxy-1,3,5-triazine (CDPhT) (0.3 g, 1 mmole) in 5 ml THF was mixed with N-methylmorpholine (0.11 ml, 1 mmole) for 30 minutes at temperature of 0-5°C to obtain N-(4,6- diphenoxy -1,3,5-triazine-2-yl)-N- methylmorpholine chloride. Then, phenyl isoserine derivative with the formula **4b**, where R₃= R₄=CH₃ (0.325 g, 1 mmol) was added and mixed for 12 hours. After N-methylmorpholine hydochloride was filtered off, and solvent was evaporated it gave respective triazine ester of the formula **6b**, where R₃= R₄=CH₃, and R₅=R₆= OC₆H₅, with the quantitative yield.
TLC: eluent CHCl₃, R_{f}=0.65
¹H-NMR (CDCl₃): δ=1.76 (s, 3H); 1.79 (s, 3H); 5.19 (d, J=8.0 Hz, 1H); 5.27 (d, J= 8.0 Hz, 1H); 7.19-7.22 (m, 2H); 7.40-7.74 (m, 18 H) ppm]
IR (KBr): 1780 [cm⁻¹]

The triazine ester, in the presence of magnesium bromide and pyrolidinpyridine, was coupled with protected Baccatin III of the generalized formula 3, where R₂=Si(C₂H₅)₃. The product was chromatographically and spectrally identical to that described in the 11. Example.

### 13. Example

Solution of 2,4-dichlor-1,3,5-triazine (DCMT) (0.09 g, 0.5 mmole) in 5 ml THF was mixed with N-methylmorpholine (0.11 ml, 1 mmole) for 30 minutes at temperature of 0-5°C to form respective quaternary triazinammonium salt. Then, phenyl isoserine derivative with the formula **4b,** where R₃= R₄=CH₃ (0.325 g, 1 mmol) was added and mixed for 12 hours, first at temperature of 0-5°C next at room temperature. N-methylmorpholine hydrochloride was filtered off, and solvent from filtrate was evaporated. It gave respective triazine ester of the formula **6b,** where R₃= R₄= CH₃, and R₅=OCH₃, with the quantitative yield.
TLC: eluent CHCl₃, R_{f}=0.60
¹H-NMR (CDCl₃): δ=1.76 (s, 6H); 1.79 (s, 6H); 3.76 (s, 3H); 5.27 (d, J= 7.5 Hz, 2H); 5.49, (d, J= 7.5 Hz, 2H); 7.20-7.22 (m, 2H); 7.43-7.56 (m, 14H); 7.43-7.70 (m, 4H) [ppm]
IR (KBr): 1800 [cm⁻¹]

The triazine ester, in the presence of pyrolidinpyridine, was coupled with protected Baccatin III of the generalized formula **3,** where R₂=Si(C₂H₅)₃. The product was chromatographically and spectrally identical to that described in the 11. Example.

### 14. Example

Solution of 2-chlor-4,6-dimethoxy-1,3,5-triazine (0.175 g, 1 mmole) in 5 ml THF was mixed with N-methylmorpholine (0.11 ml, 1 mmole) for 30 minutes at temperature of 0-5°C, to form respective quaternary triazinilammonium salt. Then, phenyl isoserine of the formula **4c** (0.267 g, 1 mmol) was added and mixed for 12 hours, first at temperature of 0-5°C, next at room temperature. N-methylmorpholine hydrochloride was filtered off and solvent was removed from the filtrate. It gave respective triazine ester of the formula 6c, where R₅=R₆=OCH₃, with the quantitative yield.
TLC: eluent CHCl₃, R_{f}=0.50
¹H-NMR (CDCl₃): δ=3,76 (s, 6H); 5.31 (d, J=7.5 Hz, 1H); 5.56 (d, J=7.5 Hz, 1H); 7.35-7.48 (m, 6H); 7.75-7.77 (m, 2H); 8.04 (d, J=8.0 Hz; 2H)[ppm]
IR (KBr): 1785 [cm⁻¹]

The triazine ester, in the presence of DMAP, was coupled with protected Baccatin III of the generalized formula **3**, where R₂=Si(C₂H₅)₃. It gave 0.885 g of the final product (yield 91.5%) with the formula 2, where R₁ is defined by the formula 9, R₂ = Si(C₂H₅)₃.
TLC: eluent (acetone:benzene 1:4), R_{f}=0.75
¹H-NMR (CDCl₃): δ= 0.57 (m, 6H); 0.95 (t, J= 8.0 Hz, 9H); 1.21 (s, 3H); 1.23 (s, 3H); 1.67 (s, 3H); 1.75 (s, 1H); 1.86-1.89 (m, 1H); 1.99 (s, 3H); 2.08 (s, 3H); 2.17 (s, 3H); 2.23-2.26 (m, 1H); 2.35-2.37 (m, 1 H); 2.54-2.57 (m, 1H); 3.84 (d, J=7.5 Hz, 1H); 4.16 (d, J=8.5 Hz, 1H); 4.31 (d, J=8.5 Hz, 1H); 4.47-4.50 (m, 1H); 4.93-4.96 (m, 2H); 5. 60 (d, J=6.5 Hz, 1 H); 5.67 (d, J=7.5 Hz, 1H); 6.15-6.19 (m, 1H); 6.43 (s, 1H); 7.35-7.39 (m, 5H); 7.47-7.51 (m, 4H); 7.59 (t, J=7.5 Hz, 1H); 7.64 (t, J= 7.5 Hz, 1 H); 8.08 (d. J=7.5 Hz, 2H); 8.25 (d, J= 7.5 Hz, 2H) [ppm]

The product of condensation was treated with 0.1 N HCl in methanol, and the final product was chromatographically purified on silica gel with the system of hexane: ethyl acetate 1.5:1. It gave 0.594 g (i.e. yield 76%) of a product spectroscopically and chromatographically identical to Paclitaxel.

### 15. Example

Solution of 2-chlor-4,6-diphenoxy-1,3,5-triazine (0.3 g, 1 mmole) in 5 ml THF was mixed with N-methylmorpholine (0.11 ml, 1 mmole) for 30 minutes at temperature of 0-5°C to obtain N-(4,6- diphenoxy -1,3,5-triazine-2-yl)-N- methylmorpholine chloride. Then, phenyl isoserine derivative with the formula **4c,** (0.267 g, 1 mmol) was added and mixed for 12 hours. After N-methylmorpholine hydochloride was filtered off, and solvent was evaporated, it gave respective triazine ester of the formula **6c,** where R₅=R₆= OC₆H₅, with the quantitative yield.
TLC: eluent CHCl₃, R_{f}=0.65
¹H-NMR (250 MHz, CDCl₃): δ=5.31 (d, J=7.5 Hz, 1H); 5.57 (d, J=7.5 Hz, 1H); 7.12-7.50 (m, 18H); 8.13 (d, J=8.0 Hz, 2H) [ppm]
IR (KBr): 1790 [cm⁻¹]

The triazine ester, in the presence of 4-pyrolidinpyridine, was coupled with protected Baccatin III of the generalized formula 3, where R₂=Si(C₂H₅)₃. The product was chromatographically and spectrally identical to that described in the 14. Example.

### 16. Example

Solution of 2,4-dichlor-6-methoxy-1,3,5-triazine (0.09 g, 0.5 mmole) in 5 ml THF was mixed with N-methylmorpholine (0.11 ml, 1 mmole) for 30 minutes at temperature of 0-5°C to obtain respective quaternary triazinammonium salt. Then, phenyl isoserine derivative with the formula **4c**, (0.267 g, 1 mmol) was added and mixed for 12 hours, first at temperature of 0-5°C, next at room temperature. After N-methylmorpholine hydochloride was filtered off, and solvent was evaporated, it gave respective triazine ester of the formula **6c**, where R₅ = OC₆H₅, with the quantitative yield.
TLC: eluent CHCl₃, R_{f}=0.55
¹H-NMR (CDCl₃): δ=3,76 (s, 3H); 5.31 (d, J=7.5 Hz, 2H); 5.56 (d, J=7.5 Hz, 2H); 7.35-7.47 (m, 12H); 7.75-7.77 (m, 4H); 8.04 (d, J=8.0 Hz; 4H)[ppm]
IR (KBr): 1785 [cm⁻¹]

The triazine ester was coupled with protected Baccatin III of generalized formula **3,** where R₂=Si(C₂H₅)₃, in the presence of magnesium bromide and diazobicycloundekane. It gave product chromatographically and spectrally identical to that of the 14 Example.

### 17. Example

Solution of 2-chlor-4,6-methoxy-1,3,5-triazine (0.175 g, 1 mmole) in 5 ml THF was mixed with N-methylmorpholine (0.11 ml, 1 mmole) for 30 minutes at temperature of 0-5°C to obtain respective quaternary triazinammonium salt. Then, phenyl isoserine derivative with the formula **4a,** where R₂ = H (0.267 g, 1 mmol) was added and mixed for 12 hours, first at temperature of 0-5°C, next at room temperature. After N-methylmorpholine hydochloride was filtered off, and solvent was evaporated, it gave respective triazine ester of the formula **6a**, where R₂=H, R₅ = OC₆H₅, with the quantitative yield.
TLC: eluent CHCl₃, R_{f}=0.4
¹H-NMR (CDCl₃): δ= δ=3,86 (s, 6H); 5.41 (d, J=7.8 Hz, 1 H); 5.72 (d, J=7.8 Hz, 1H); 7.00-7.38 (m, 8H); 7.44 (d, J=8.0 Hz; 2H)[ppm]
IR (KBr): 1795 [cm⁻¹]

For 5 days the triasine ester, in the presence of 4-(N,N-dimethylamino)pyridine and magnesium bromide was coupled with protected Baccatin III of the generalized formula 3, where R₂=Si(C₂H₅)₃. It gave 0.800 g (yield 81%) of the product characterized by R_{f}=0.65 (eluent acetone: benzene 1:4), which without purification was treated with concentrated hydrochloric acid in tetrahydrofurane for 10 min. After purification on silica gel in a chromatographic column in the mixture of hexane and ethyl acetate 1:1 as the movable phase, it was obtained 0.482 g (yield 70%) of the product spectroscopically and chromatographically identical to Paclitaxel described in the 1. Example.

## Claims

1. The method for production of semi-finished products useful in synthesis of Paclitaxel, of the generalized formula 2, 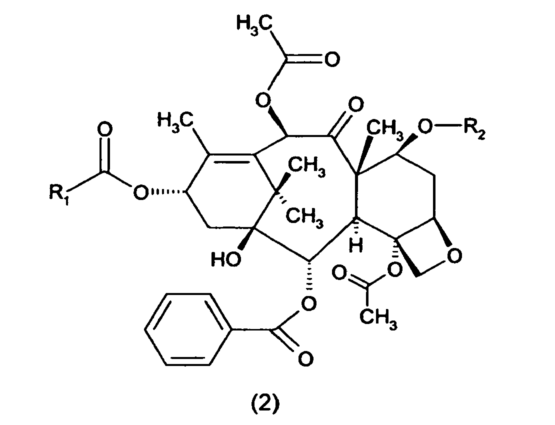 where R₁ denotes the group with the formula 7, 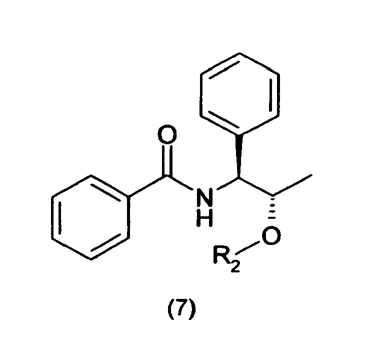 or with the formula 8, 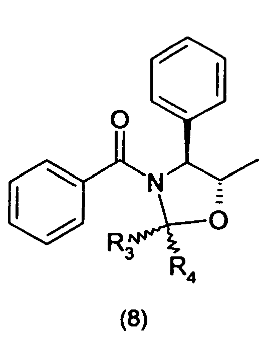 or with the formula 9, 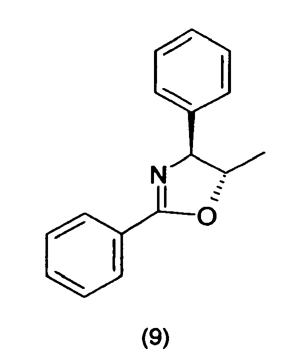 or with the formula 10, 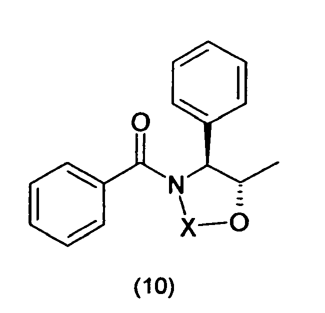 R₂ denotes hydrogen, alkylsilol group or substituted alkylsilol group, arylsilol group or substituted arylsilol group, alkylarylsilol group or substituted alkylarylsilol group, alkoxycarbonyl group or substituted alkoxycarbonyl group, alkoxymethyl group or substituted alkoxymethyl group, R₃ and R₄ denote hydrogen, alkyl group, substituted alkyl group, aryl group, substituted aryl group, whereas X denotes C=O, C=S, S=O, SO₂, (C=O)₂, P(aryl)₃, P-N(alkyl)₂, (P=O)-N(alkyl)₂, (P=S)-N(alkyl)₂ groups, by reaction of Baccatin III derivative and phenyl isoserine derivative, **characterized in that** phenyl isoserine derivatives with generalized formulas 4a-d, 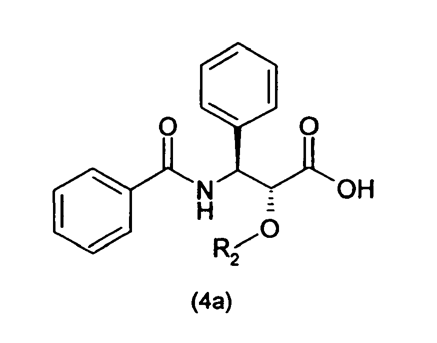 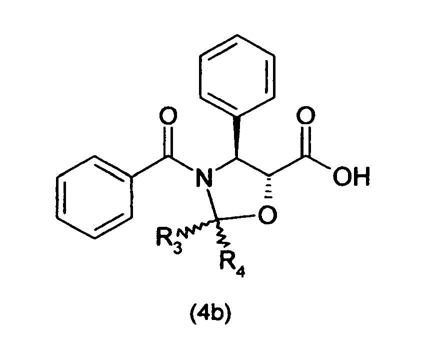 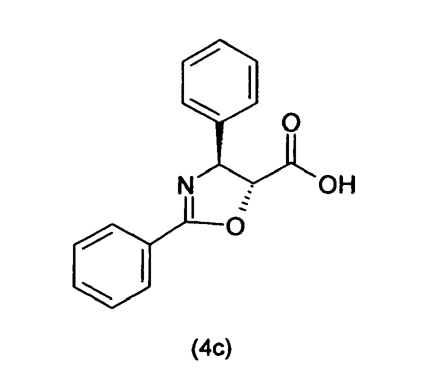 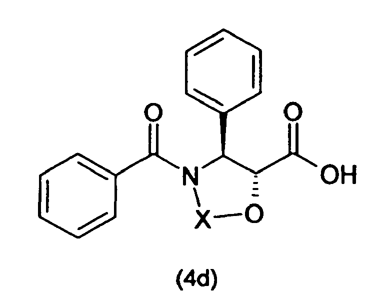 in which R₂, R₃, R₄ and X have above specified meanings, or mixtures of their epimerides with different configurations on the carbon 2' or their salts are treated with triazine condensing agent having the generalized formula 5, 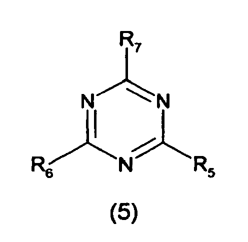 in which R₅ and R₆ denote chlorine, alkyloxylic group, substituted alkyloxylic group, aryloxylic group, substituted aryloxylic group, whereas R₇ denotes fluorine, chlorine or quaternary ammonium group, possibly in the presence of anhydrous organic solvent, next so obtained triazine esters of phenyl isoserine with generalized formulas 6a-d, 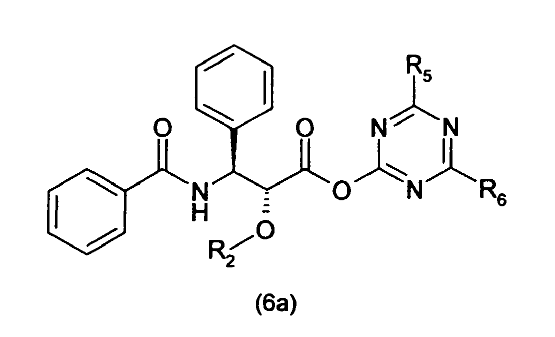 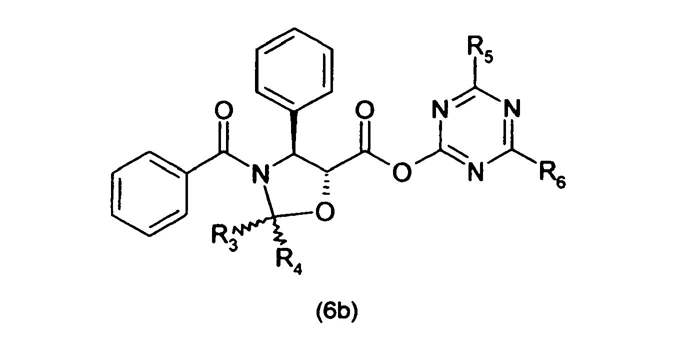 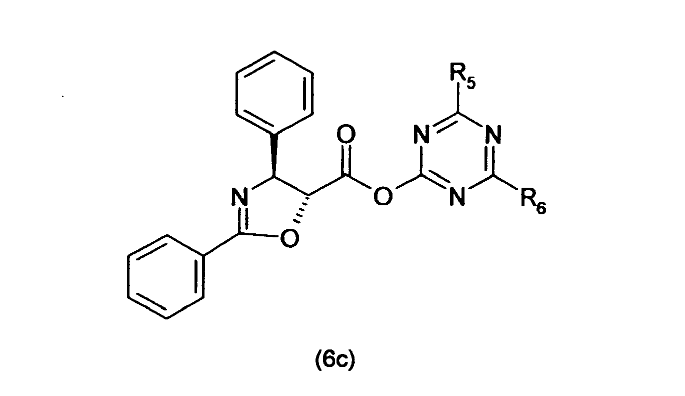 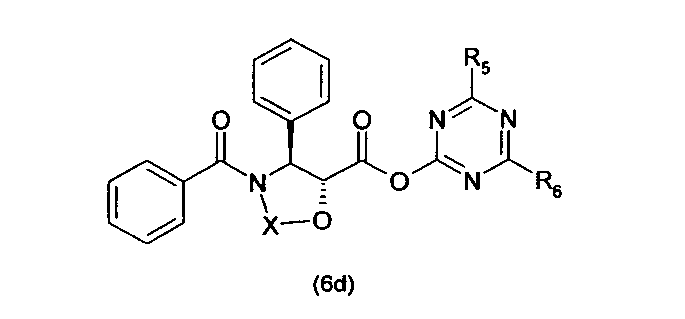 in which R₂, R₃, R₄ and X have above specified meanings, is reacted with Baccatin III, with the generalized formula 3, 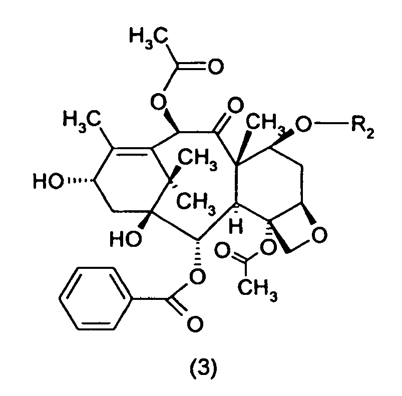 where R₂ denotes hydrogen or a group which protects the hydroxyl group in the position 7, in an anhydrous organic solvent, in the presence of a catalyst if required.

2. The method according to claim 1, **characterized in that** the groups protecting the hydroxyl function in the 7 position of Baccatin there are used alkylsilol group or substituted alkylsilol group, arylsilol group or substituted arylsilol group, alkylarylsilol group or substituted alkylarylsilol group, alkoxycarbonyl group or substituted alkoxycarbonyl group, alkoxymethyl group or substituted alkoxymethyl group.

3. The method according to claim 2, **characterized in that** as the group protecting the hydroxyl function in the 7. Position of Baccatin there is used triethylsilil group.

4. The method according to claim 1, **characterized in that** as the salts of phenyl isoserine derivatives metal salts are used, particularly alkali metal salts or tertiary amine salts, preferable of N-methylmorfoline.

5. The method according to claim 1, **characterized in that** as triazin based condensing agent 2-chloro-4,6-dimethoxy-1,3,5-triazine is used.

6. The method according to claim 1, **characterized in that** as triazin based condensing agent quaternary triazinilammonium salt is used, obtained by treatment of tertiary amine with 2-chlor-4,6-dimethoxy-1,3,5-triazine.

7. The method according to claim 1, **characterized in that** as tertiary amine N-methylmorpholine, tetramethylguanidyne, 1,4-dimethylpiperazyne, N-methylopyrolidyne, N,N'-tetramethyloethyldiamine, N-methylpiperydyne, pyridine, pyridine derivatives substituted in the carbon atom in the pyridine ring or diazabicycloundekane are used.

8. The method according to claim 1, **characterized in that** triazin esters with the generalized formulas 6a-d 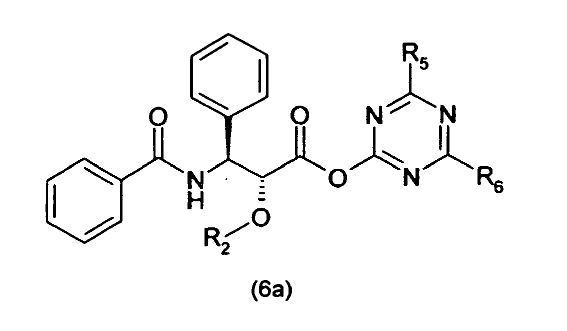 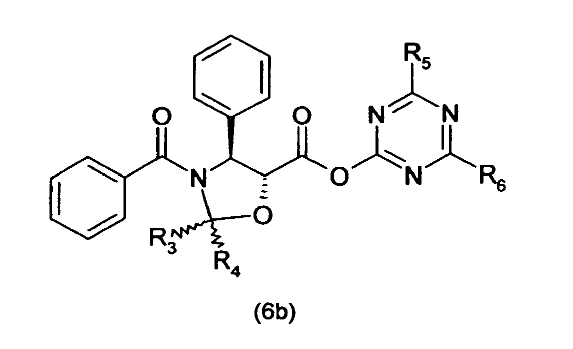 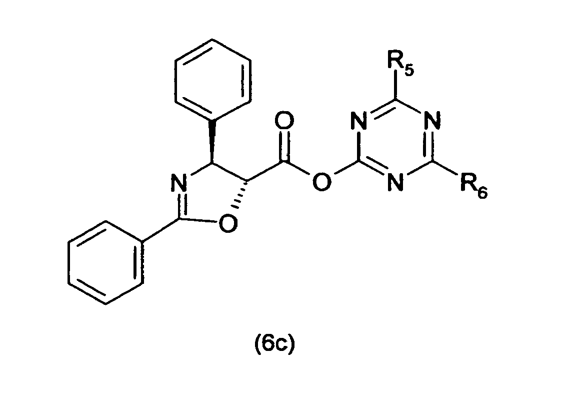 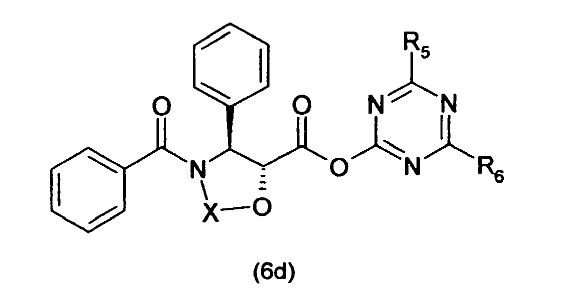 are removed from the reaction mixture before treatment with Baccatin derivatives.

9. The method according to claim 1, **characterized in that** reaction with Baccatin is accomplished in the presence of a Lewis type acid.

10. The method according to claim 9, **characterized in that** the Lewis type acid is anhydrous magnesium bromide.

11. The method according to claim 1, **characterized in that** reaction with Baccatin is accomplished in the presence of a base.

12. The method according to claim 11, **characterized in that** as the base tertiary amine is used.

13. The method according to claim 11, **characterized in that** as the tertiary amine 4-(N,N-dimethylamino)pyridine or 4-(pyrolidin-1)pyridine or 1,8-diazabicyclo[5.4.0.]undec-7-en are used.

14. The method according to claim 1, **characterized in that** reaction with Baccatin is accomplished with use of anhydrous magnesium bromide in the presence of 4-(N,N-dimethylamino)pyridine or 4-(pyrolidyno-1)pyridine or 1,8-diazabicyclo[5.4.0.]undec-7-ene.

15. The method according to claim 1, **characterized in that** as solvent a-proton solvent is used.

16. The method according to claim 15, **characterized in that** as solvent aromatic hydrocarbons, chlorinated hydrocarbons, ethers, esters, acidamides, acidonitriles are used.

17. The method according to claim 16, **characterized in that** as solvent toluene, dichloromethane, dichloroethane, tetrahydrofuran, ethyl acetate, N,N-dimethyloformamide, N,N-dimethyloacetamide and phosphoric acid N,N',N'-hexamethyloamide, acetonitryle are used.

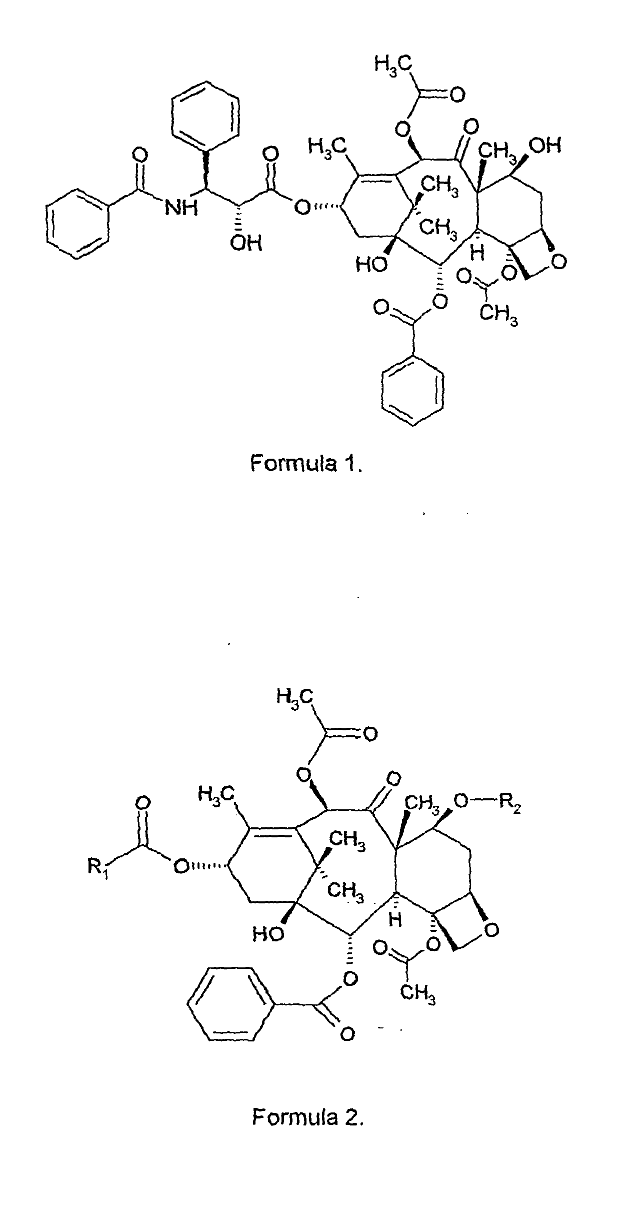

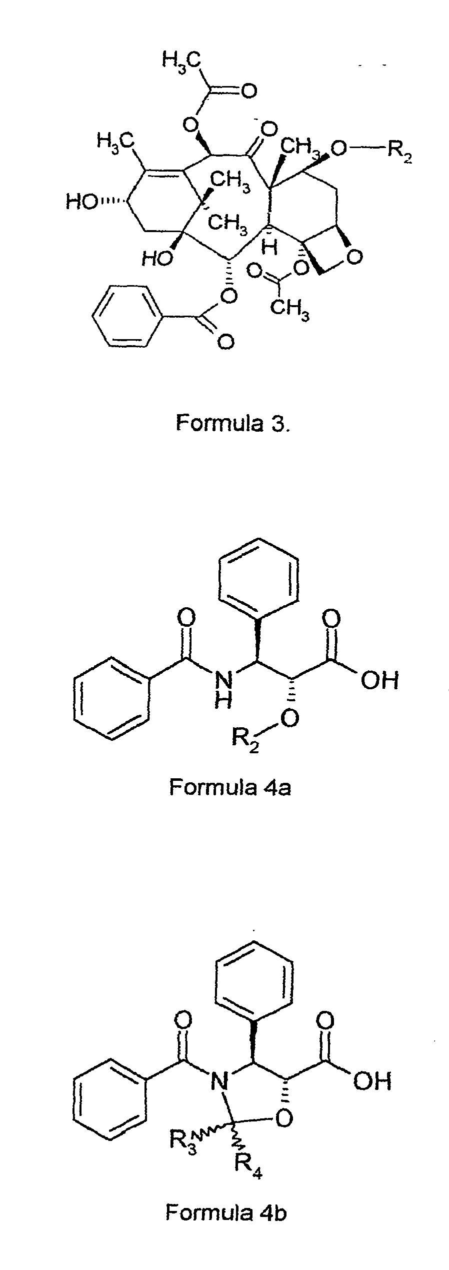

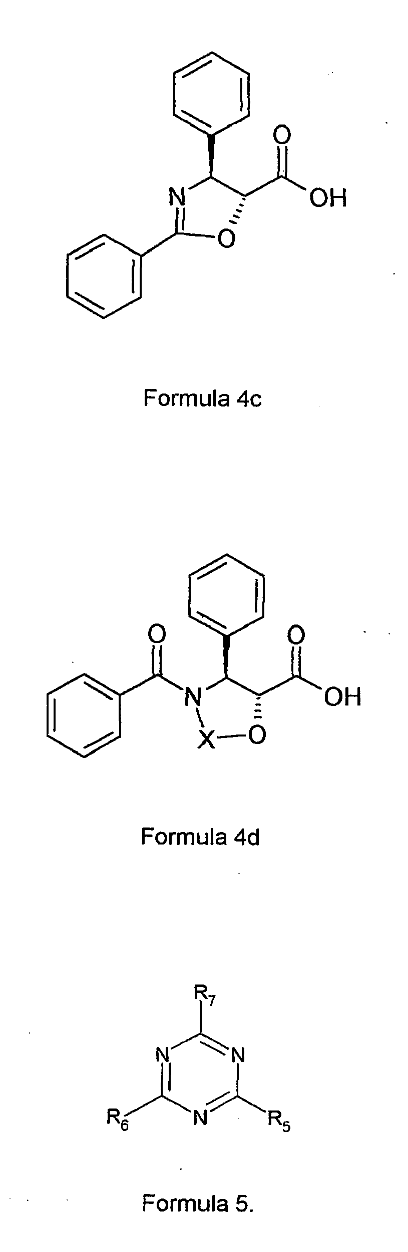

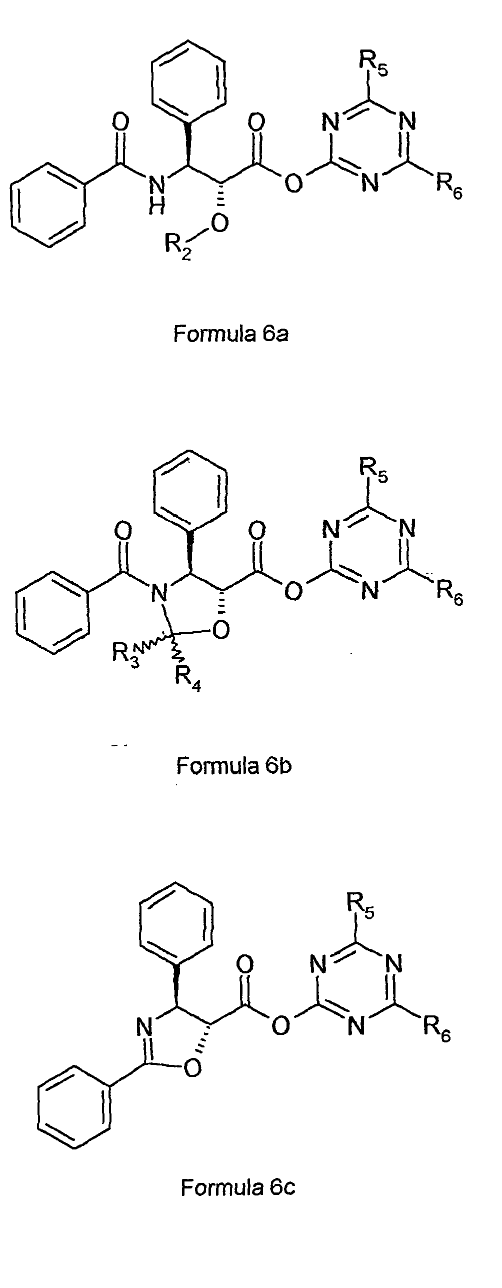

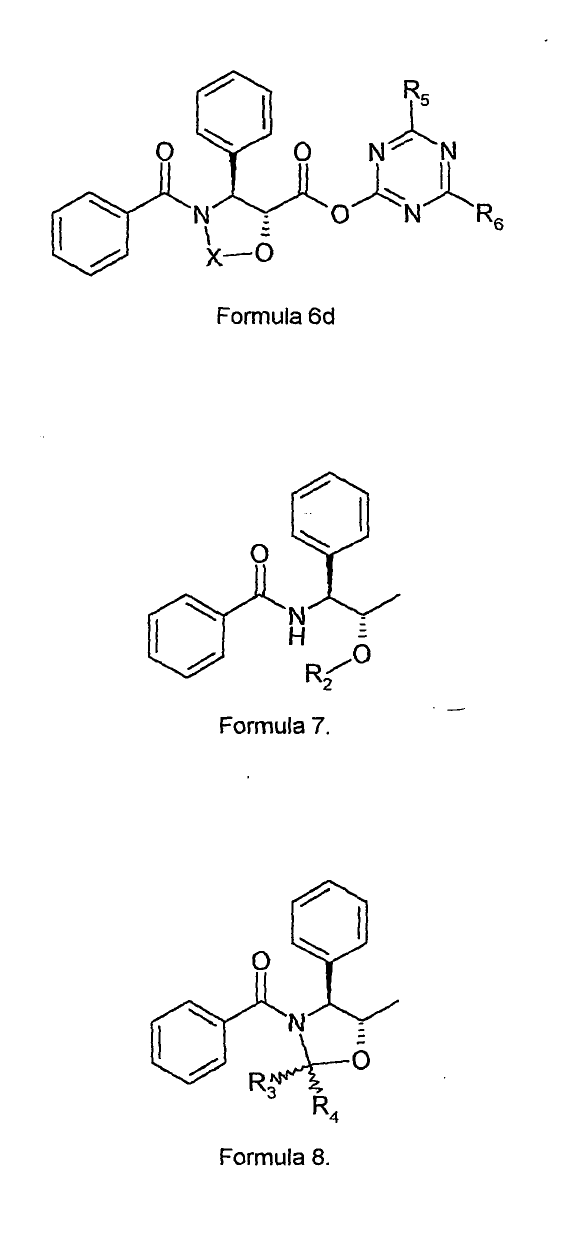

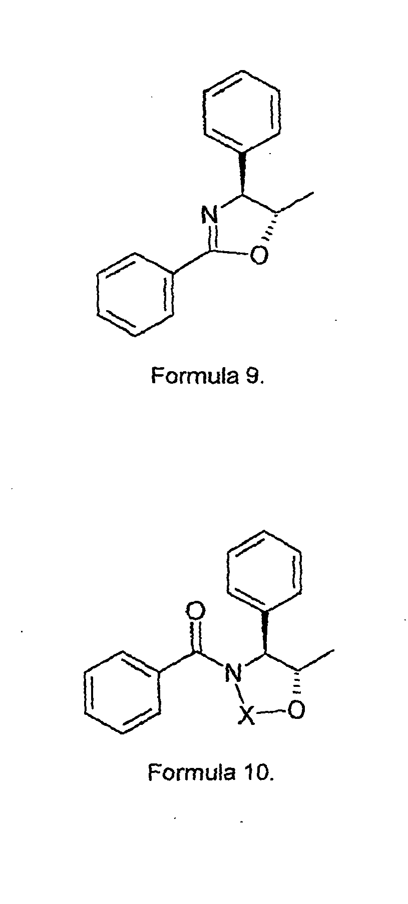

## Patentansprüche

1. Verfahren zur Herstellung von Halbfertigen Produkten verwendbar für die Synthese des Paclitaxels, mit der allgemeinen Formel 2, 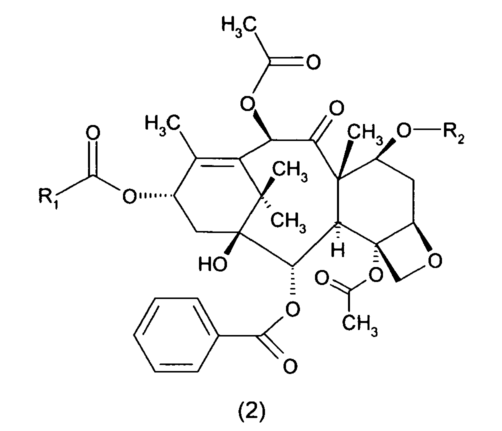 in der R₁ die Gruppe mit der Formel 7 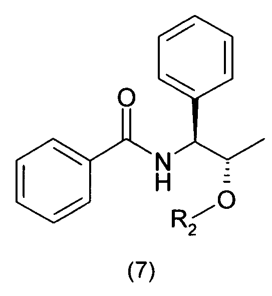 oder die Gruppe mit der Formel 8 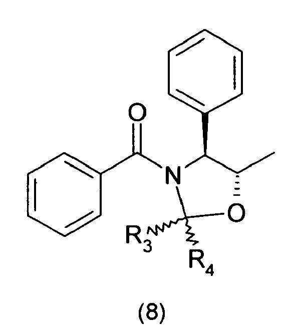 oder die Gruppe mit der Formel 9 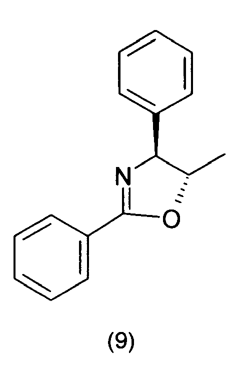 oder die Gruppe mit der Formel 10 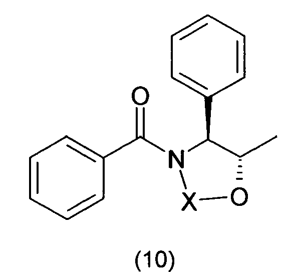 bedeutet, R₂ Wasserstoff, Alkylsilyl- Gruppe oder substituierte Alkylsilyl - Gruppe, Arylosilyl - Gruppe oder substituierte Arylosilyl - Gruppe, Alkyloarylosil - Gruppe oder substituierte Alkyloarylosil - Gruppe, Alkoxykarbonylgruppe oder substituierte Alkoxykarbonylgruppe, Alkoxymethylengruppe oder substituierte Alkoxymethylengruppe bedeutet, R₃ i R₄ Wasserstoff, Alkylgruppe, substituierte Alkylgruppe, Arylgruppe, substituierte Arylgruppe bedeutet, und X Gruppe C=O, C=S, S=O, SO₂, (C=O)₂, P(aryl)₃, P-N(alkyl)₂ , (P=O)-N(alkyl)₂, (P=S)-N(alkyl)₂ bedeutet, in der Reaktion Bakatinderivate III und Phenyloisoserin **dadurch gekennzeichnet, dass** Phenyloisoserinderivate mit den allgemeinen Formeln 4a -d, 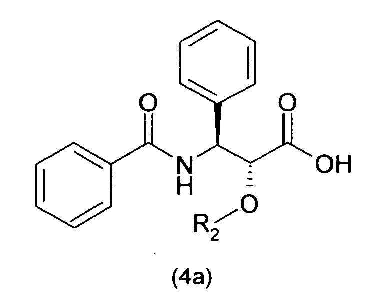 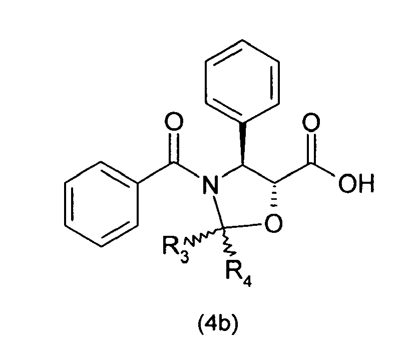 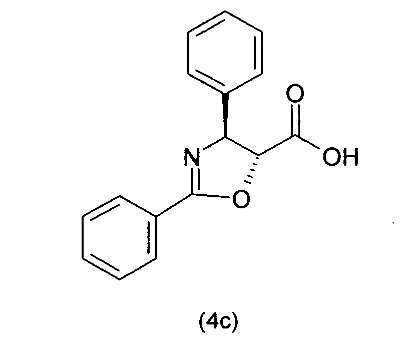 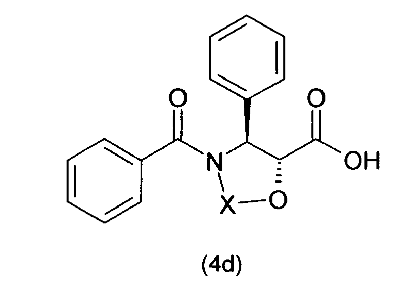 in den R₂, R₃, R₄ und X, die oben genannte Bedeutung haben oder Gemisch ihrer Epimer mit differenzierten Konfigurationen auf Kohle 2' oder ihre Salze einer Reaktion mit dem kondensierenden Triazinreagenz mit der allgemeinen Formel 5 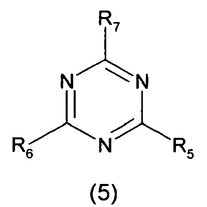 unterzogen werden, in dem R₅ und R₆ Chlor, Alkyloxylgruppe, substituierte Alkyloxylgruppe, Aryloxylgruppe oder substituierte Aryloxylgruppe bedeuten, dagegen R₇ Fluor, Chlor oder Quartäramongruppe bedeutet, eventuell im Medium des wasserfreien, organischen Lösungsmittel, und daraufhin so gewonnene Triazinester des Phenyloisoserins mit der allgemeinen Formel 6a - d, 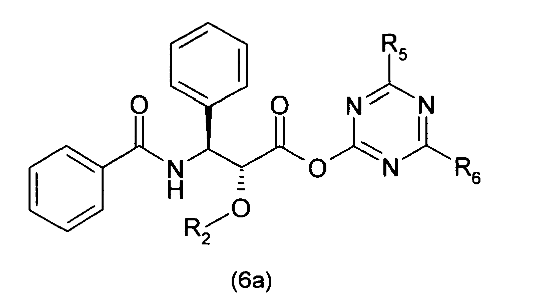 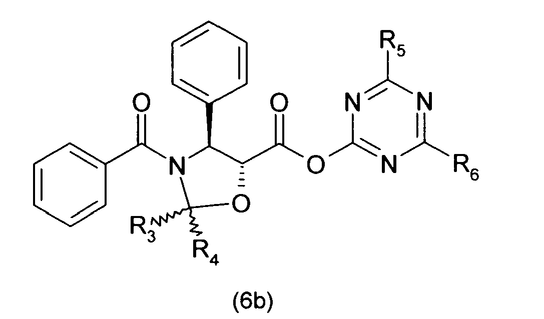 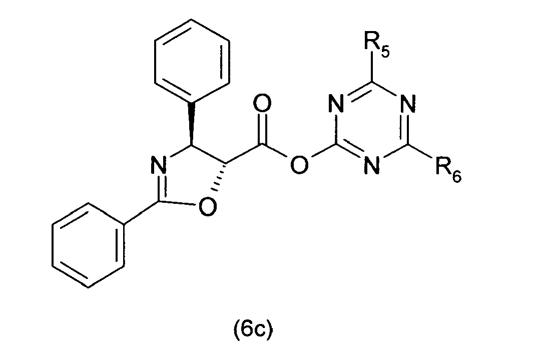 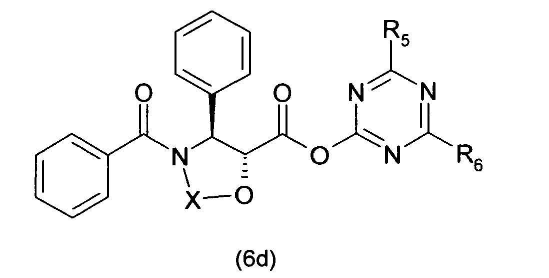 in den R₂, R₃, R₄ und X die oben genannte Bedeutung haben, einer Reaktion mit Bakatin III mit der allgemeinen Formel 3, 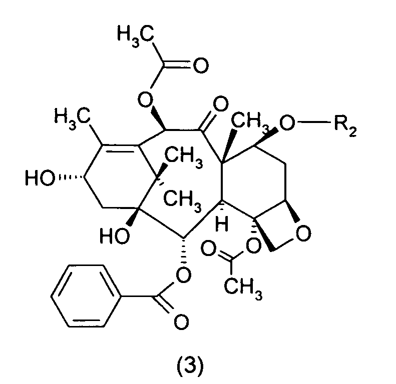 in der R₂ Wasserstoff oder eine Gruppe, die die Hydroxylfunktion in der Position 7 sichert, im dem wasserfreien, organischen Lösungsmittel, eventuell in Gegenwart von Katalysator, unterzogen werden

2. Das Verfahren nach Anspruch 1, **dadurch gekennzeichnet, dass** man als die Gruppen, die die Hydroxylfunktion in der Position 7 von Bakatin sichern, die Alkylsilyl - Gruppe oder substituierte Alkylsilyl Gruppe, Arylosilyl - Gruppe oder substituierte Arylosilyl - Gruppe, Alkyloarylosil - Gruppe oder substituierte Alkyloarylosil - Gruppe, Alkoxykarbonylgruppe oder substituierte Alkoxykarbonylgruppe, Alkoxymethylengruppe oder substituierte Alkoxymethylengruppe verwendet.

3. Das Verfahren nach Anspruch 2, **dadurch gekennzeichnet, dass** man als Gruppe, die die Hydroxylfunktion in der Position 7 von Bakatin sichert, Trietylosilyl -Gruppe verwendet.

4. Das Verfahren nach Anspruch 1, **dadurch gekennzeichnet , dass** man als Salze der Phenolisoserinsalzderivate die Metallsalze verwendet, insbesondere der alkalischen Metalle oder Salze des tertiären Amins, vorteilhaft N - Methylmorphin.

5. Das Verfahren nach Anspruch 1, **dadurch gekennzeichnet, dass** man als Triazinkondensationsmittel 2-Chlor-4,6 Dimethoxy -1,3,5- Triazin verwendet.

6. Das Verfahren nach Anspruch 1, **dadurch gekennzeichnet, dass** man als Triazinkondensationsmittel Quartärtriazinammonsalz, gewonnenes durch die Wirkung von 2-Chlor-4,6 Dimethoxy -1,3,5- Triazin auf das tertiäre Amin, verwendet.

7. Das Verfahren nach Anspruch 1, **dadurch gekennzeichnet , dass** man als tertiäres Amin N-Methylomorpholin, Tetramethyloguanidin, 1,4 Diamethylpiperazin, N-Methylpyrolydin, N,N'-Tetramethyläthyldiamin, N- Methylpyridin, Pyridin, Pyridinderivate substituiert auf dem Kohlenatom im Pyridinring , Diazabicycloundecan verwendet.

8. Das Verfahren nach Anspruch 1, **dadurch gekennzeichnet, dass** Triazinester des Phenylisoserin mit der allgemeinen Formel 6a -d 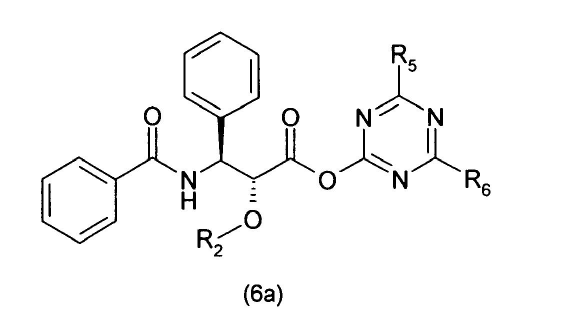 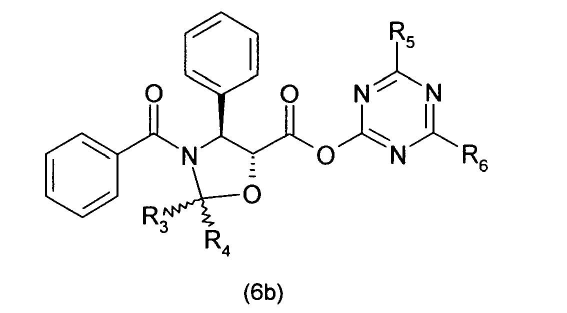 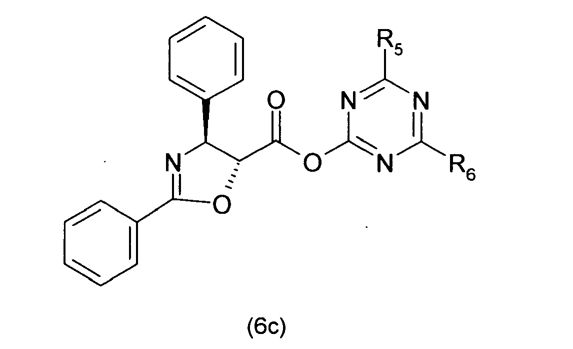 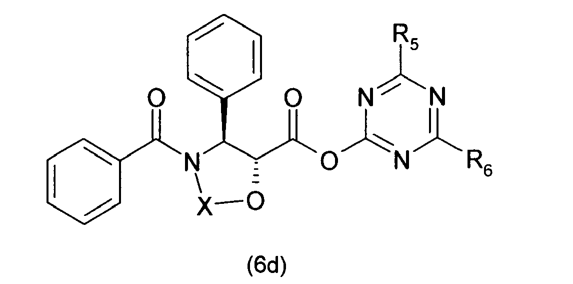 aus dem Reaktionsmedium vor ihrer Reaktion mit den Bakatinderivaten ausgesondert werden.

9. Das Verfahren nach Anspruch 1, **dadurch gekennzeichnet, dass** die Reaktion mit Bakatin in Anwesenheit von Lewis- säure durchgeführt wird.

10. Das Verfahren nach Anspruch 9, **dadurch gekennzeichnet, dass** als Lewis - säure wasserfreies Magnesiumbromid verewendet wird.

11. Das Verfahren nach Anspruch 1, **dadurch gekennzeichnet, dass** die Reaktion mit Bakatin in Gegenwart von Base durchgeführt wird.

12. Das Verfahren nach Anspruch 11, **dadurch gekennzeichnet , dass** als Base ein tertiäres Amin verwendet wird.

13. Das Verfahren nach Anspruch 12, **dadurch gekennzeichnet, dass** 4- (Diamethylamin) Pyridin oder 4- (Pyrolydin-1) Pyridin oder 1,8 Diazabicyclo [5.4.0.]Undec-7-en als das tertiäre Amin verwendet wird.

14. Das Verfahren nach Anspruch 12, **dadurch gekennzeichnet, dass** die Reaktion mit Bakatin mit dem wasserfreien Magnesiumbromid in Anwesenheit von 4- (Diamethylamin) Pyridin oder 4-(Pyrolydin- 1) Pyridin oder 1,8 Diazabicyclo [5.4.0.)Undec-7-en durchgeführt wird.

15. Das Verfahren nach Anspruch 1, **dadurch gekennzeichnet, dass** ein aprotisches Lösungsmittel als Lösungsmittel verwendet wird.

16. Das Verfahren nach Anspruch 15, **dadurch gekennzeichnet, dass** aromatische Kohlenwasserstoffe, halogenisierte Kohlenwasserstoffe, Äther, Ester, Säureamide, Säurenitril als Lösungsmittel verwendet werden.

17. Das Verfahren nach Anspruch 16, **dadurch gekennzeichnet, dass** Toluol, Dichloromethan, Dichloräthan, Tetrahydrofuran, Essigsäureäthyl, N,N - Dimethylformamid, N,N- Dimethylacetamid und N,N',N'- Hexamethylamid der Phosphorsäure, Azetonitril als Lösungsmittel verwendet werden.

## Revendications

1. Procédé de production de produits semi-finis utiles dans la synthèse du paclitaxel, à la formule chimique générale 2, 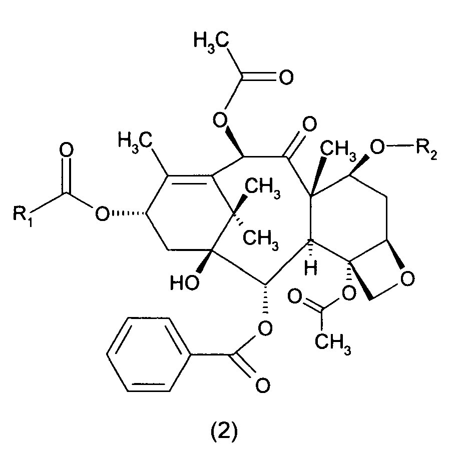 dans laquelle R₁ désigne un groupement de formule 7, 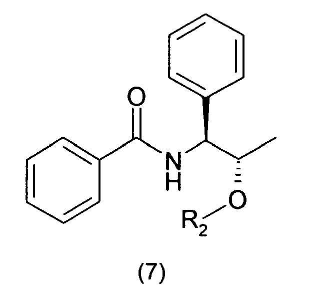 ou un groupement de formule 8, 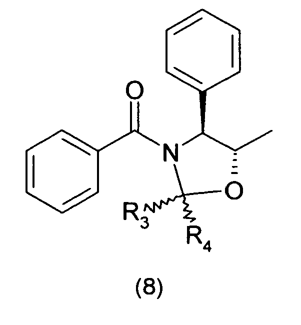 ou un groupement de formule 9, 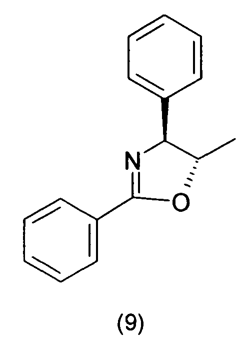 ou un groupement de formule 10, 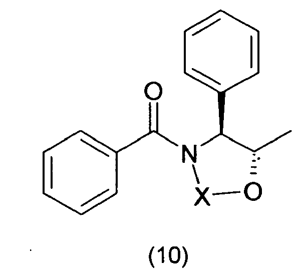 R₂ désigne l'hydrogène, un groupement alkylo-silyle ou un groupement alkylo-silyle substitué, un groupement arylo-silyle ou un goupement arylo-silyle substitué, un groupement alkylo-arylo-silyle ou un groupement alkylo-arylo-silyle substitué, un groupement alkoxy-carbonyle ou un groupement alkoxy-carbonyle substitué, un groupement alkoxy-méthyle ou un groupement alkoxy-méthyle substitué, R₃ et R₄ désignent l'hydrogène, un groupement alkyle, un groupement alkyle substitué, un groupement aryle, un groupement aryle substitué, et X désigne un groupement C=O, C=S, S=O, SO₂, (C=O)₂, P(aryl)₃, P-N(alkyl)₂, (P=O)-N(alkyl)₂, (P=S)-N(alkyl)₂, par réaction d'un dérivé de baccatine III et d'un dérivé de phénylisoserine, **caractérisé en ce que** les dérivés de phénylisoserine aux formules générales 4a-d, 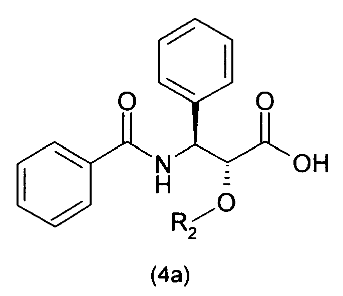 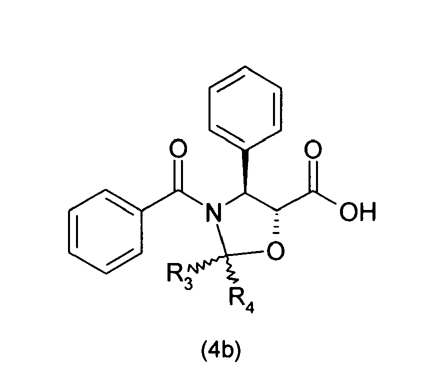 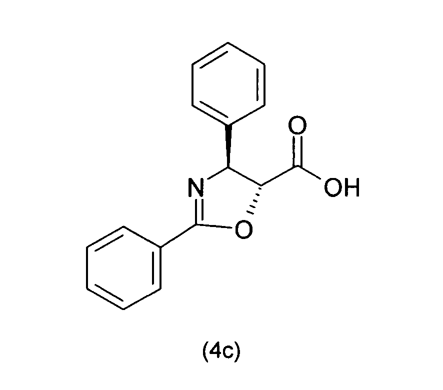 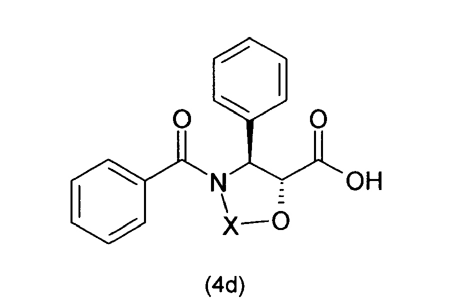 dans lesquelles R₂, R₃, R₄ et X ont la signification mentionnée ci-dessus, ou les mélanges de leurs épimères de configurations variées sur carbone 2' ou leurs sels, sont soumis à une réaction avec un réactif de condensation à triazine de formule générale 5, 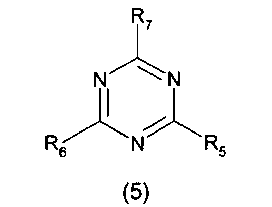 où R₅ et R₆ désigne le chlore, un groupement alkyloxy, un groupement alkyloxy substitué, un groupement aryloxy ou un groupement aryloxy substitué, tandis que R₇
désigne le fluor, le chlore ou un groupement ammonium quaternaire, éventuellement en présence de solvant organique anhydre, puis les esters triazines de phénylisoserine ainsi obtenus, aux formules générales 6a-d, 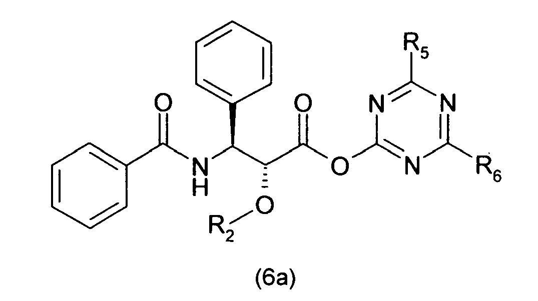 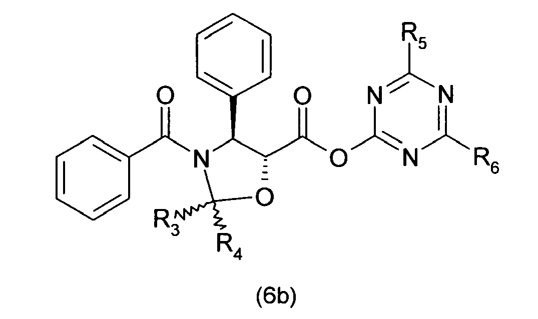 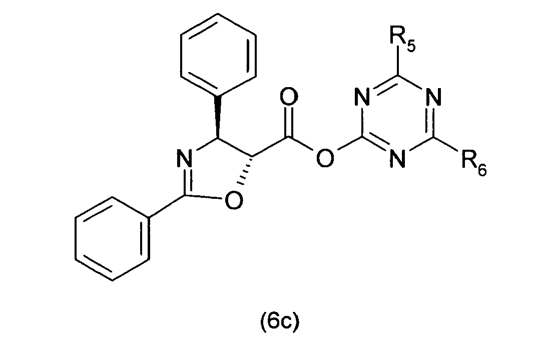 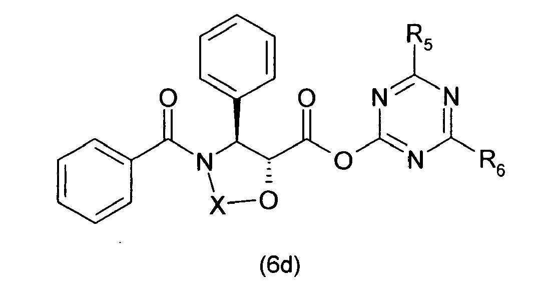 dans lesquelles R₂, R₃, R₄ et X ont la signification mentionnée ci-dessus, sont soumis à la réaction avec la baccatine III à la formule générale 3, 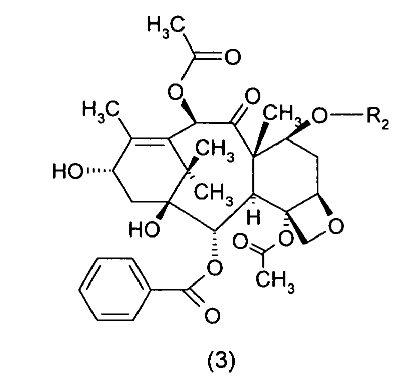 dans laquelle R₂ désigne l'hydrogène ou un groupement protecteur de la fonction hydroxy en position 7, dans un solvant organique anhydre, éventuellement en présence d'un catalyseur.

2. Procédé selon la revendication 1 **caractérisé en ce que**, en tant que groupement protecteur de la fonction hydroxy en position 7 de la baccatine, est utilisé un groupement alkylsilyle ou un groupement alkylsilyle substitué, un groupement arylsilyle ou un groupement arylsilyle substitué, un groupement alkyloarylosilyle ou un groupement alkyloarylosilyle substitué, un groupement alkoxycarbonyle ou un groupement alkoxycarbonyle substitué, un groupement alkoxyméthyle ou un groupement alkoxyméthyle substitué.

3. Procédé selon la revendication 2 **caractérisé en ce que**, en tant que groupement protecteur de la fonction hydroxy en position 7 de la baccatine, est utilisé un groupement triéthylsilyle.

4. Procédé selon la revendication 1 **caractérisé en ce que**, en tant que sels de dérivés de la phénylisoserine, sont utilisés des sels de métaux, et surtout de métaux alcalins ou des sels d'amines tertiaires, avantageusement de la N-méthylmorpholine.

5. Procédé selon la revendication 1 **caractérisé en ce que**, en tant que réactif de condensation à triazine, est utilisée la 2-chloro-4,6-diméthoxy-1,3,5-triazine.

6. Procédé selon la revendication 1 **caractérisé en ce que**, en tant que réactif de condensation à triazine, est utilisé le sel quaternaire triazinyl-ammonium obtenu par action de la 2-chloro-4,6-diméthoxy-1,3,5-triazine sur une amine tertiaire.

7. Procédé selon la revendication 1 **caractérisé en ce que**, en tant qu'amine tertiaire, est utilisée la N-méthylmorpholine, la tétraméthylguanidine, la 1,4-diméthylpipérazine, la N-méthylpyrolidine, la N,N'-tétraméthyléthylènediamine, la N-méthylpipéridine, la pyridine, des dérivés de la pyridine en substitution sur un atome de carbone en cycle pyridine, le diazabicycloundecane.

8. Procédé selon la revendication 1 **caractérisé en ce que** les esters triazines de phénylisoserine aux formules générales 6a-d 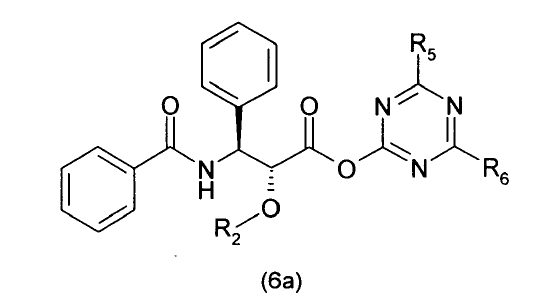 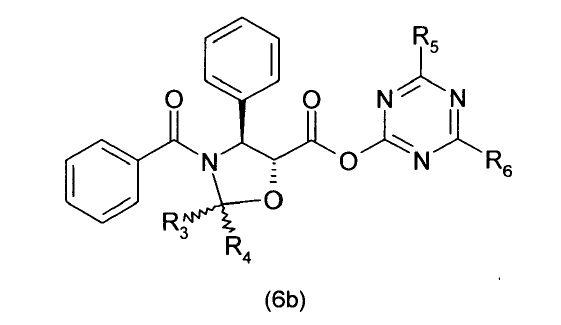 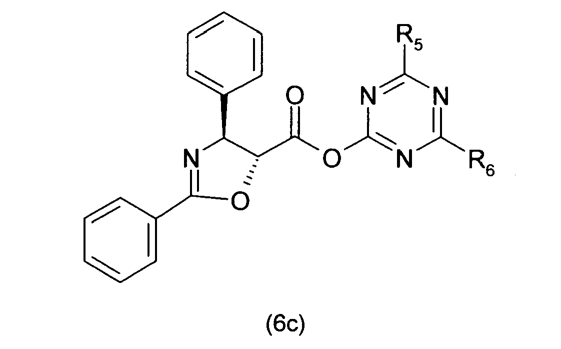 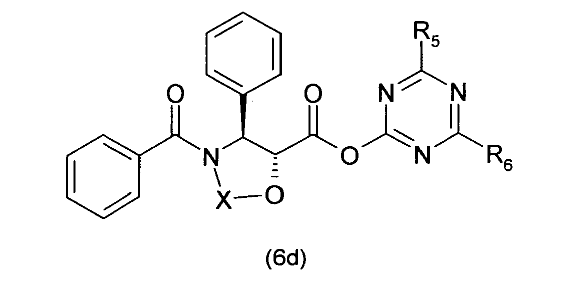 sont isolés du milieu de la réaction avant d'être soumis à la réaction avec les dérivés de baccatine.

9. Procédé selon la revendication 1 **caractérisé en ce que** la réaction avec la baccatine est menée en présence d'un acide de Lewis.

10. Procédé selon la revendication 9 **caractérisé en ce que** le bromure de magnésium anhydre est utilisé en tant qu'acide de Lewis.

11. Procédé selon la revendication 1 **caractérisé en ce que** la réaction avec la baccatine est menée en présence d'une base.

12. Procédé selon la revendication 11 **caractérisé en ce que** la base utilisée est une amine tertiaire.

13. Procédé selon la revendication 12 **caractérisé en ce que** l'amine tertiaire utilisée est la 4-(N,N-diméthylamino)pyridine ou la 4-(pyrrolidyno-1)pyridine ou du 1,8-diazabicyclo[5.4.0.]undec-7-ène.

14. Procédé selon la revendication 1 **caractérisé en ce que** la réaction avec la baccatine est menée avec utilisation du bromure de magnésium anhydre en présence de la 4-(N,N-diméthylamino)pyridine ou la 4-(pyrrolidyno-1)pyridine ou du 1,8-diazabicyclo[5.4.0.]undec-7-ène.

15. Procédé selon la revendication 1 **caractérisé en ce que** le solvant utilisé est un solvant aprotique.

16. Procédé selon la revendication 15 **caractérisé en ce que** le solvant utilisé est un hydrocarbure aromatique, un hydrocarbure halogéné, un éther, un ester, une amide d'acide, un nitrile d'acide.

17. Procédé selon la revendication 16 **caractérisé en ce que** le solvant utilisé est le toluène, le dichlorométhane, le dichloroéthane, le tétrahydrofurane, l'acétate éthylique, le N,N-diméthylformamide, le N,N-diméthylacétamide et le N,N',N'-hexaméthylphosphoramide, l'acétonitrile.
